# EUROPEAN PATENT SPECIFICATION

(11) **EP 2 758 071 B1**
(45) Date of publication and mention of the grant of the patent: **28.11.2018**
(21) Application number: 12772616.4
(22) Date of filing: 21.09.2012
(51) Int. Cl.: A61K 39/00, A61P 25/00

(54) **VACCINE THERAPY**
IMPFSTOFFTHERAPIE
VACCINOTHÉRAPIE

(30) Priority: 23.09.2011 WO PCT/US2011/052992
(43) Date of publication of application: 30.07.2014
(73) Proprietor: AC Immune S.A., 1015 Lausanne (CH)
(72) Inventor: PFEIFER, Andrea, 1806 St-Legier (CH); MUHS, Andreas, 1053 Cugy (CH); MADANI, Rime, 1006 Lausanne (CH); BELICHENKO, Pavel Vasilyevich, San Diego, CA 92111 (US); MOBLEY, William Charles, La Jolla, CA 92037 (US)
(74) Representative: Vossius & Partner Patentanwälte Rechtsanwälte mbB
(86) International application number: PCT/US2012/056728
(87) International publication number: WO 2013/044147

(56) References cited:
- WO-A2-2007/068411
- WO-A2-2010/106127
- STOLTZNER S E ET AL: "TEMPORAL ACCRUAL OF COMPLEMENT PROTEINS IN AMYLOID PLAQUES IN DOWN'S SYNDROME WITH ALZHEIMER'S DISEASE", AMERICAN JOURNAL OF PATHOLOGY; [10640], AMERICAN SOCIETY FOR INVESTIGATIVE PATHOLOGY, US, vol. 156, no. 2, 1 February 2000 (2000-02-01), pages 489-499, XP001146406, ISSN: 0002-9440 cited in the application
- ROIZEN NANCY J: "Complementary and alternative therapies for Down syndrome.", MENTAL RETARDATION AND DEVELOPMENTAL DISABILITIES RESEARCH REVIEWS 2005, vol. 11, no. 2, 2005, pages 149-155, XP002689521, ISSN: 1080-4013

## Description

The present invention provides means for treating amyloid-related pathology in young to middle aged subjects with Down's syndrome (DS). In particular, the present invention provides antigenic peptide fragments derived from amyloid protein or amyloid-like protein for use in the treatment of Alzheimer's disease (AD)-like dementia in young to middle aged subjects with Down's syndrome (DS).

The genetic disorder Down's syndrome (DS) is the most common trisomy and it occurs in one out of 700-1000 newborns. Estimates suggest that 25% or more of individuals with Down syndrome over age 35 show the signs and symptoms of Alzheimer's-like dementia (Stanton L.R and Coetzee R.H , 2004). The percentage increases with age. In DS, the entire or at least a part of the chromosome 21 is present in triplicate (Antonarakis *et al.,* 2004; Moncaster *et al.,* 2010). Consequently, the three copies of the gene of the amyloid precursor protein (APP) lead to the generation of an excess of Amyloid-β (Aβ), one of the main abnormal proteins well known to be responsible for Alzheimer's disease (AD) (Ballard *et al.,* 2011). Therefore, Aβ is suggested to have a possible role for the AD-like dementia in most DS people (Lee *et al.,* 2005;Liu *et al.,* 2005;Gilman *et al.,* 2005).

In people with Down syndrome, AD-like memory defects can be related to several pathological proteins, such as Aβ, Tau, Dyrk1A, Apo E etc. Aβ increase begins already in the embryonic stage, progresses at birth and continues to build up with increasing age (Stoltzner *et al.,* 2000). In addition, Cerebrospinal fluid (CSF) levels of Aβ42 were lower and tau levels were higher in older (>40 years) than in younger DS people (Tapiola *et al.,* 2001). Once Aβ is deposited in plaques, Apo E can be detected in many plaques at age 12 and augments steadily with age (Lemere *et al.,* 1996). Neurofibrillary tangles (NFTs) are detected in the brain of DS during the fourth decade of life. These NFT are believed to result from tau accumulation. It has been shown that hyperphosphorylation might be caused by overexpression of a kinase named Dyrk1A (dual-specificity tyrosine-phosphorylated and regulated kinase 1A) (Lemere *et al.,* 1996;Liu *et al.,* 2008). Therefore, people with DS can develop amyloid-related pathology by age 40 and most have the clinical symptoms similar to Alzheimer's, such as cognitive decline and memory impairment, by age 60 (Stanton 2004).

While individuals with DS receive medical care mainly for their various health problems (such as heart defect, infections and hypothyroidism), specific-treatment for neuropathological traits, i.e. mental disability and memory deficiency, is rarely considered. Currently, only few clinical trials are ongoing, all aimed to enhance mental abilities or to reduce nerve damage. The drugs for treating DS are also used in AD and all act on the cholinergic system or the glutamatergic neurotransmission such as Rivastigmine or Donepezil, a cholinesterase inhibitors and Memantine, a NMDA receptor antagonist (Prasher, 1993; Prasher, 2004). However, evidence of efficacy is lacking for people with DS. Currently, there are many amyloid-modifying treatments under review, including targeting-Aβ-immunotherapy (Rafii, 2010). Several vaccines have recently reached clinical phases (Weiner and Frenkel, 2006) after having shown efficient reduction of cerebral Aβ burden and reversing cognitive decline in mouse AD models. In contrast to AD, immunotherapies targeting Aβ are not being addressed in DS.

Of the several approaches for treating cognitive impairment in adults with DS, existing studies using acetylcholinesterase inhibitors (AChEls) or NMDA receptor antagonists have shown little or no effect (Fernandez et al., 2007, Hanney et al., 2012). In contrast, there is significant support for the hypothesis that amyloid plays a role in the cognitive *deterioration* of people with DS; Netzer and colleagues showed that altering APP cleavage, by a γ-secretase inhibitor, has a *beneficial* effect on the memory of a DS mouse model (Netzer et al., 2010). These observations build on studies in mouse models showing that increased gene dose for APP, the parent protein of the Abeta peptide responsible for amyloid deposition, is necessary for the age-linked degeneration of neurons that characterizes DS and AD (Salehi et al 2006, 2009). Therefore, targeting amyloid can be a promising therapeutic strategy for the prevention of AD progression. The development of an immunotherapy against Aβ is based on the hypothesis that if a molecule targets and sequesters Aβ (in soluble form and as oligomers) *in situ,* the molecule can enhance removal of Abeta from the brain and bring clinical benefits to people with DS. It is anticipated that the antibodies generated by the anti- Aβ vaccine will bind to fibrillar Aβ deposits to eventually solubilize or inhibit the growth of the pre-plaques. The Aβ oligomers are today considered as the most toxic Aβ species, which impairs most cognitive functions in DS people (Teller et al., 1996).
WO 2007/068411 A2 discloses methods and compositions for the therapeutic and diagnostic use in the treatment of diseases and disorders which are caused by or associated with amyloid or amyloid-like proteins. In particular, WO 2007/068411 A2 discloses an antigenic construct comprising an Aβ antigenic peptide fragment consisting of a single or repetitive stretch of between 13 and 15 contiguous amino acid residues from the N-terminal part of the Aβ peptide reconstituted in a liposome for use in the treatment of an amyloid- associated disease or condition.
WO 2010/106127 A2 discloses an antigenic composition comprising an antigen derived from an amyloid protein or amyloid-like protein such as, for example amyloid beta, prion protein, tau protein, alpha-synuclein, huntingtin, which is modified by a lipophilic or hydrophobic moiety that facilitates insertion into the lipid bilayer of a liposome for inducing a T-cell independent immune response upon treatment of a disease, condition or disorder in a patient in need of such a T-cell independent response.

There is therefore a need for a prevention therapy which aims at preventing or slowing down the development of clinical symptoms associated with amyloid-related pathology in subjects with Down's syndrome (DS), such as, particularly, memory and/or cognitive impairments or abnormalities.

In particular, there is a need for a treatment of clinical symptoms associated with amyloid-related pathology in subjects with Down's syndrome (DS), , which leads to an improvement in learning capabilities in subjects with Down's syndrome (DS) and/or improvement or restoration of memory and/or cognitive capacities.

Within the scope of the present invention the measures and means are provided which help to meet this need. In particular, the present invention provides antigenic peptides for use in prevention therapy and in the treatment of AD-like cognitive impairments in children and in young to middle-aged subjects with Down's syndrome.
The present invention is defined by the claims and the following embodiments:
In a first embodiment, the present invention relates to an antigenic peptide derived from amyloid beta protein for use in the treatment and/or alleviation and/or prevention of memory and/or cognitive impairments or abnormalities in subjects with Down's syndrome, wherein the antigenic peptide consists of residues Aβ1-15 and is modified by a palmitic acid bound to the N- and/or C-terminus of the peptide and reconstituted in a liposome and said subjects with Down's syndrome have not yet developed amyloid protein-associated plaques in the brain.
In a specific embodiment, the Aβ1-15 peptide is modified by four palmitic acids bound to the N- and/or C-terminus of the peptide.
In another specific embodiment the Aβ1-15 peptide is modified by two palmitic acids bound to the N- and/or C-terminus of the peptide.
In various other embodiments, the invention relates to the antigenic peptide for use according to any one of embodiments 1-3, wherein said impairment or abnormality concerns an impairment of recognition memory, an impairment of contextual associative memory, an impairment in associative learning, an impairment of declarative memory for facts and events, episodic memory impairments, a language dysfunction, a visuospatial impairment, a decrease in executive functions, personality changes, emotional changes, apraxia, impairments of performing learned motor tasks, pyramidal and extrapyramidal findings as well as myoclonus or seizures, or a combination thereof.
In still other embodiments, the invention relates to the antigenic peptide for use according to any one of the preceding embodiment, wherein said subject with Down's syndrome is
(a) a young to middle-aged subject; or
(b) a middle-aged subject; or
(c) a young-aged subject; or
(d) a children-aged subject, or,
(e) a subject below age 65.
In various further embodiments, the invention relates to the antigenic peptide for use according to any one of the preceding embodiments, wherein said use
a) leads to an amelioration and/or restoration of memory; and/or
b) leads to an increase in the retention or a complete restoration of cognitive capacity in the treated subject; and/or
c) leads to enhancing or restoring memory and/or cognitive capacity; and/or
d) does not induce unwanted side effects.
In a specific embodiment, the invention relates to the antigenic peptide for use according to any one of the preceding embodiments for prevention of the development of Aβ-associated plaques in the brain.
In another embodiment, the invention relates to a composition comprising the antigenic peptide as defined in any one of the preceding embodiments in a therapeutically effective amount together with a pharmaceutically acceptable carrier and/or excipient for use in the treatment and/or alleviation and/or prevention of memory and/or cognitive impairments or abnormalities in subjects with Down's syndrome, wherein said subjects with Down's syndrome have not yet developed amyloid protein-associated plaques in the brain.
In various specific embodiments, the invention relates to the composition for use according to the preceding embodiment, comprising an adjuvant, particularly wherein the adjuvant is lipid A, particularly wherein the adjuvant is a detoxified lipid A, particularly wherein the adjuvant is monophosphoryl or diphosphoryl lipid A, alum, calcium phosphate, interleukin 1, and/or microcapsules of polysaccharides and proteins.
In another specific embodiment, the present invention relates to the antigenic peptide for use according to any one of the preceding embodiments or to the composition for use according to any one of the preceding embodiments, for prevention of neurodegeneration in a subject with Down's syndrome.
In one embodiment, the present invention relates to the antigenic peptide for use according to any one of the preceding embodiments or to the composition for use according to any one of the preceding embodiments, for inducing an immune response against amyloid beta protein in a subject with Down's syndrome.

Disclosed herein is an antigenic peptide fragment derived from amyloid protein or amyloid-like protein, particularly an antigenic peptide fragment derived from amyloid-beta protein, or a composition comprising said antigenic peptide, for use in the treatment and/or alleviation and/or prevention of memory and/or cognitive impairments or abnormalities, particularly of AD-like memory and/or cognitive impairments or abnormalities, particularly impairments and abnormalities originating in the hippocampus and/or the prefrontal cortex and/or the entorhinal cortex of the brain, in young to middle-aged subjects with Down's syndrome, in particular in young subjects with Down's syndrome, who have not yet developed amyloid protein- or amyloid-like protein-associated plaques, particularly Aβ-associated plaques, in the brain.

Further disclosed herein is an antigenic peptide fragment derived from amyloid protein or amyloid-like protein, particularly an antigenic peptide fragment derived from amyloid beta protein, or a composition comprising said antigenic peptide, for use in the treatment, and/or alleviation and/or prevention of memory and/or cognitive impairments or abnormalities, particularly of AD-like memory and/or cognitive impairments or abnormalities, particularly impairments and abnormalities originating in the hippocampus and/or the prefrontal cortex and/or the entorhinal cortex of the brain, in middle-aged subjects with Down's syndrome, in particular in middle-aged subjects with Down's syndrome, who have not yet developed amyloid protein- or amyloid-like protein-associated, particularly Aβ-associated, plaques in the brain.

Further disclosed herein is an antigenic peptide fragment derived from amyloid protein or amyloid-like protein, particularly derived from amyloid beta protein, or a composition comprising said antigenic peptide, for use in the treatment, and/or alleviation and/or prevention of memory and/or cognitive impairments or abnormalities, particularly of AD-like memory and/or cognitive impairments or abnormalities, particularly impairments and abnormalities originating in the hippocampus and/or the prefrontal cortex and/or the entorhinal cortex of the brain, in young subjects with Down's syndrome, in particular in young subjects with Down's syndrome, who have not yet developed amyloid protein- or amyloid-like protein-associated, particularly Aβ-associated-, plaques in the brain.

Further disclosed herein is an antigenic peptide fragment derived from amyloid protein or amyloid-like protein, particularly derived from amyloid beta protein, or a composition comprising said antigenic peptide, for use in the treatment, and/or alleviation and/or prevention of memory and/or cognitive impairments or abnormalities, particularly of AD-like memory and/or cognitive impairments or abnormalities, particularly impairments and abnormalities originating in the hippocampus and/or the prefrontal cortex and/or the entorhinal cortex of the brain, in childrens with Down's syndrome, in particular in children subjects with Down's syndrome, who have not yet developed amyloid protein- or amyloid-like protein-associated, particularly Aβ-associated, plaques in the brain.

Further disclosed herein is an antigenic peptide fragment derived from amyloid protein or amyloid-like protein, particularly derived from amyloid beta protein, or a composition comprising said antigenic peptide, for use in prevention of memory and/or cognitive impairments or abnormalities, particularly of AD-like memory and/or cognitive impairments or abnormalities, particularly impairments and abnormalities originating in the hippocampus and/or the prefrontal cortex and/or the entorhinal cortex of the brain, in children and in young to middle-aged subjects with Down's syndrome, in particular in young subjects with Down's syndrome, who have not yet developed amyloid protein- or amyloid-like protein-associated, particularly Aβ-associated plaques in the brain.

In particular, the antigenic peptide is administered to a subject with Down's syndrome, particularly a subject with Down's syndrome suffering from memory and/or cognitive impairments or abnormalities, particularly of AD-like memory and/or cognitive impairments or abnormalities, particularly impairments and abnormalities originating in the hippocampus and/or the prefrontal cortex and/or the entorhinal cortex of the brain, wherein said subject is below age 60, particularly below age 55, particularly below age 50, particularly below age 45, particularly below age 40, particularly below age 35, particularly below age 30, particularly below 25, particularly below age 20, particularly below age 15, particularly below age 10, particularly below age 5, particularly below age 3;
or the antigenic peptide is administered to a subject with Down's syndrome, particularly a subject with Down's syndrome suffering from memory and/or cognitive impairments or abnormalities, particularly of AD-like memory and/or cognitive impairments or abnormalities, particularly impairments and abnormalities originating in the hippocampus and/or the prefrontal cortex and/or the entorhinal cortex of the brain, wherein said subject is below age 35, particularly below age 30, particularly below 25, particularly below age 20, particularly below age 15;
or the antigenic peptide is administered to a subject with Down's syndrome, particularly a subject with Down's syndrome suffering from memory and/or cognitive impairments or abnormalities, particularly of AD-like memory and/or cognitive impairments or abnormalities, particularly impairments and abnormalities originating in the hippocampus and/or the prefrontal cortex and/or the entorhinal cortex of the brain, wherein said subject is below age 15, particularly below age 10, particularly below age 5, particularly below age 3;
or the antigenic peptide is administered to a subject with Down's syndrome, particularly a subject with Down's syndrome suffering from memory and/or cognitive impairments or abnormalities, particularly of AD-like memory and/or cognitive impairments or abnormalities, particularly impairments and abnormalities originating in the hippocampus and/or the prefrontal cortex and/or the entorhinal cortex of the brain, wherein said subject is below age 10, particularly below age 5, particularly between 0 and 10, particularly between 1 and 10, particularly between 2 and 10, particularly between 3 and 10, particularly between 4 and 10, particularly between 5 and 10.

Treatment with the antigenic peptide as disclosed herein prevents the development of Aβ-associated plaques in the brain, particularly in the hippocampus and/or the prefrontal cortex and/or the entorhinal cortex of the brain.

It is further contemplated herein using the antigenic peptide fragment derived from amyloid protein or amyloid-like protein, or a composition comprising said antigenic peptide according to the invention and as described herein in the treatment, and/or alleviation and/or prevention of memory and/or cognitive impairments or abnormalities, particularly of AD-like memory and/or cognitive impairments or abnormalities, particularly impairments and abnormalities originating in the hippocampus and/or the prefrontal cortex and/or the entorhinal cortex of the brain, in children and in young to middle-aged subjects with Down's syndrome who have already developed Aβ-associated plaques in the brain.

Treatment with the antigenic peptide as provided in the present invention reduces the amount of Aβ-associated plaques in the brain, particularly in the hippocampus and/or the prefrontal cortex and/or the entorhinal cortex of the brain.

The patient group to be treated with the antigenic peptide or the composition as disclosed herein may comprise young to middle-aged subjects with Down's Syndrome, particularly subjects, which are below the age of 65, particularly below the age of 60, 55 or 50. In one embodiment, subjects with an age of between 0 and 65, particularly of between 5 and 55, particularly of between 10 and 50, particularly of between 15 and 45, particularly of between 20 and 40, particularly of between 25 and 35, may be treated with the antigenic peptide or the composition according to the invention.

For the purpose of the present invention, children refer to subjects who are below the age of 18 particularly subjects with an age of between 0 and 18 particularly of between 1 and 10, particularly of between 2 and 9, particularly between 3 and 9, particularly of between 4 and 9, particularly between 5 and 18.

For the purpose of the present invention, young subjects refer to subjects who are below the age of 35, particularly subjects with an age of between 0 and 35 particularly of between 1 and 30, particularly of between 5 and 25.

For the purpose of the present invention, middle-aged subjects refer to subjects with an age of between 36 and 65 particularly of between 40 and 60, particularly of between 45 and 55.

Treatment of a children or young to middle-aged subject with Down's syndrome and suffering from memory and/or cognitive impairments or abnormalities, particularly from AD-like memory and/or cognitive impairments or abnormalities, particularly impairments and abnormalities originating in the hippocampus and/or the prefrontal cortex and/or the entorhinal cortex of the brain, using the antigenic peptide or the composition as described herein, leads to an amelioration or restoration of said impairments or abnormalities, particularly to memory amelioration, particularly amelioration or restoration of the recognition memory and/or the contextual associative memory.

In particular, treatment of a children or young to middle-aged subject with Down's syndrome and suffering from cognitive impairments or abnormalities, particularly from AD-like memory and/or cognitive impairments or abnormalities, using the antigenic peptide or the composition as described herein, leads to an increase in the retention or a complete restoration of cognitive memory capacity in the treated subject.

The treatment of AD-like cognitive impairments or abnormalities in children and young to middle-aged subjects with Down's syndrome with the antigenic peptide as described herein shows the therapeutic effects as disclosed herein without inducing unwanted side effects such as meningoencephalitis and microhemorrhage.

The subject may be a children-aged subject, particularly a young-aged subject, particularly a middle-aged subject as defined herein, with Down's syndrome and suffering from memory and/or cognitive impairments or abnormalities, particularly from AD-like memory and/or cognitive impairments or abnormalities, particularly impairments and abnormalities originating in the hippocampus and/or the prefrontal cortex and/or the entorhinal cortex of the brain.

The treatment with the antigenic peptide or the composition as described herein, prevents the development of Aβ-associated plaques in the brain, particularly in the hippocampus and/or the prefrontal cortex and/or the entorhinal cortex of the brain, in subjects with Down's syndrome, particularly in the brain of young- to middle-aged, particularly in the brain of children-aged, particularly in the brain of young-aged, particularly in the brain of middle-aged subjects, with Down's syndrome.

The antigenic peptide as described herein may be derived from an amyloid protein or amyloid-like protein selected from the group consisting of prion protein, tau protein, alpha-synuclein, huntingtin and amyloid-β or a combination of one or more of the above peptides.

Said Aβ antigenic peptide fragment may corespond to the N-terminal part of the Aβ peptide, particularly to the N-terminal part comprising at least 5, particularly at least 6, particularly at least 7, particularly at least 8, particularly at least 9, particularly at least 10, particularly at least 11, particularly at least 12, particularly at least 13, particularly at least 14, particularly all, amino acid residues from the Aβ1-15 fragment.

The Aβ antigenic peptide fragment may correspond to the N-terminal part of the Aβ peptide comprising at least 5, particularly at least 6, particularly at least 7, particularly at least 8, particularly at least 9, particularly at least 10, particularly at least 11, particularly at least 12, particularly at least 13, particularly at least 14, particularly at least 15, particularly all, amino acid residues from the Aβ1-16 fragment, the Aβ1-17 fragment, the Aβ1-18 fragment, the Aβ1-19 fragment, the Aβ1-20 fragment, the Aβ1-22 fragment, the Aβ1-23 fragment, the Aβ1-24 fragment, the Aβ1-25 fragment or, the Aβ1-26 fragment, or the 3-15 Aβ fragment.

In the alternative, the Aβ antigenic peptide fragment may correspond to the C-terminal part of the Aβ peptide comprising at least 5, particularly at least 6, particularly at least 7, particularly at least 8, particularly at least 9, particularly at least 10, particularly at least 11, particularly at least 12, particularly at least 13, particularly at least 14, particularly at least 15, particularly all amino acid residues from the Aβ20-40 or Aβ20-42 fragment, the Aβ21-40 or Aβ21-42 fragment, the Aβ22-40 or Aβ22-42 fragment, the Aβ23-40 or Aβ23-42 fragment, the Aβ24-40 or Aβ24-42 fragment, the Aβ25-40 or Aβ25-42 fragment, the Aβ26-46 or Aβ27-42 fragment, or the Aβ27-40 or Aβ27-42 fragment, or the Aβ29-40.

In the alternative, the Aβ antigenic peptide fragment may correspond to the middle part of the Aβ peptide comprising at least 5, particularly at least 6, particularly at least 7, particularly at least 8, particularly at least 9, particularly at least 10, particularly at least 11, particularly at least 12, particularly at least 13, particularly at least 14, particularly at least 15, particularly all amino acid residues from the Aβ15-35, particularly the Aβ20-35 fragment.

In particular, the Aβ antigenic peptide fragment may correspond to the central part of the Aβ peptide, particularly the Aβ14-29 fragment; or

the Aβ antigenic peptide fragment may correspond to the C-terminal part of the Aβ peptide, particularly the C-terminal Aβ22-35 fragment.

In the alternative, the full length Aβ1-39, Aβ1-40, or Aβ1-42 fragment may be used.

The Aβ antigenic peptide fragment as disclosed herein may contain one or more modified or non-natural amino acid residues.

Also contemplated herein is the use of Aβ antigenic peptide fragments, which are not fragments consisting of a single or repetitive stretch of between 13 and 15 contiguous amino acid residues from the N-terminal part of the Aβ peptide, particularly not fragments, wherein said contiguous stretch of 13 to 15 amino acid residues is obtained from the N-terminal fragment 1-16 or 1-17 of the Aβ peptide, particularly from the N-terminal part of the Aβ peptide selected from the group consisting of residues 1-15, 1-14, and 1-13, particularly consisting of Aβ₁₋₁₅ peptide antigen as given in SEQ ID NO: 1 and Aβ_{1-16(Δ14)} as given in SEQ ID NO: 3 disclosed in WO 2007/068411.
The antigenic peptide as described herein may be presented reconstituted in a carrier such as, for example, a vesicle, a particulate body or molecule, but particularly reconstituted in a liposome.

In particular, the antigenic peptide as described herein may be presented in a single or repetitive array on the surface of the carrier or liposome.

Said highly repetitive array on the surface of the carrier or liposome may comprise at least 10 repetitive antigenic units/carrier molecule, particularly at least 50 repetitive antigenic units/carrier molecule, particularly at least 100 repetitive antigenic units/carrier molecule, particularly at least 200 repetitive antigenic units/carrier molecule, particularly at least 300 repetitive antigenic units/carrier molecule; particularly at least 400 repetitive antigenic units/carrier molecule, particularly at least 500 repetitive antigenic units/carrier molecule.

The antigenic composition as described herein may comprise a conformational antigen, particularly an Aβ antigen as described herein, wherein more than 30%, particularly more than 40%, particularly more than 50%, particularly more than 60%, particularly more than 70%, particularly more than 80%, particularly more than 90%, particularly more than 95% and up to 100% is in a beta-sheet conformation.

In particular, the composition may comprise the antigenic peptide as described herein in a therapeutically effective amount together with a pharmaceutically acceptable carrier and/or excipient for use in the treatment, and/or alleviation and/or prevention of memory impairments or abnormalities in subjects with Down's syndrome.

In the alternative, the composition may comprise the antigenic peptide of the invention as described herein in a therapeutically effective amount together with a pharmaceutically acceptable carrier and/or excipient, for use in the treatment, and/or alleviation and/or prevention of cognitive impairments or abnormalities in subjects with Down's syndrome.

In particular said memory and/or cognitive impairments originate in the hippocampus and/or the prefrontal cortex and/or the entorhinal cortex of the brain.

The composition comprising the antigenic peptide as described herein in a therapeutically effective amount together with a pharmaceutically acceptable carrier and/or excipient may be used in the treatment and/or alleviation and/or prevention of impairments of recognition memory and/or impairments of the contextual associative memory and/or impairments of the associative learning and/or impairments of the declarative memory for facts and events and/or episodic memory impairments and/or language dysfunction such as aphasia and/or visuospatial impairments, such as misplacement of items and difficulty navigating in unfamiliar and familiar terrain and/or decreased executive functions, such as apathy, disinhibition, social isolation, poor judgment, difficulties with planning and/or poor abstract reasoning and/or personality changes and/or emotional changes such as apathy, agitation and psychosis and/or apraxia and/or impairments of performing learned motor tasks and/or other neurological signs including pyramidal and extrapyramidal findings as well as myoclonus or seizures.

In particular said impairment or abnormality concerns the recognition memory.

Said antigenic peptide for use in enhancing or restoring recognition memory in subjects with Down's syndrome may be derived from the N-terminal part of the Aβ peptide, particularly said antigenic peptide consists of all or part of amino acid residues Aβ1-15, Aβ1-16, Aβ1-17, Aβ1-18, Aβ1-19, Aβ1-20, Aβ1-22, or Aβ1-23.

In particular said antigenic peptide consists of amino acid residues Aβ1-15.

In the alternative, said antigenic peptide for use in enhancing or restoring recognition memory in subjects with Down's syndrome may be derived from the N-terminal part of the Aβ peptide, particularly said antigenic peptide consists of all or part of amino acid residues.

In particular said impairment or abnormality concerns the contextual associative memory.

In particular, said antigenic peptide for use in enhancing or restoring contextual associative memory in subjects with Down's syndrome may be derived from the N-terminal part of the Aβ peptide, particularly said antigenic peptide consists of all or part of amino acid residues Aβ1-15, Aβ1-16, Aβ1-17, Aβ1-18, Aβ1-19, Aβ1-20, Aβ1-22, or Aβ1-23.

In particular said antigenic peptide consists of amino acid residues Aβ1-15.

In particular said impairment or abnormality concerns the associative learning.

Said antigenic peptide for use in enhancing or restoring associative learning in subjects with Down's syndrome may be derived from the N-terminal part of the Aβ peptide, particularly said antigenic peptide consists of all or part of amino acid residues Aβ1-15, Aβ1-16, Aβ1-17, Aβ1-18, Aβ1-19, Aβ1-20, Aβ1-22, or Aβ1-23.

In particular said antigenic peptide consists of amino acid residues Aβ1-15.

In another alternative, said antigenic peptide for use in enhancing or restoring associative learning in subjects with Down's syndrome may be derived from the C-terminal part of the Aβ peptide, particularly said antigenic peptide consists of all or part of amino acid residues Aβ20-36, Aβ20-40, Aβ20-42, Aβ21-36, Aβ21-40, Aβ21-42, Aβ22-36, Aβ22-40 or Aβ22-42.

In particular said antigenic peptide consists of amino acid residues Aβ22-35.

The formation and stabilization of the desired conformation of the antigenic peptide may be achieved by presenting the antigenic peptide attached to, or incorporated or reconstituted, partially or fully, into a carrier such as, for example, a vesicle, a particulate body or molecule or any other means that can suitably serve as a carrier/adjuvant for the antigenic peptide. In particular,
the antigenic peptide may be attached to, or incorporated or reconstituted in the carrier through weak interactions such as, for example, van der Waal's, hydrophobic or electrostatic interaction, or a combination of two or more of said interactions, such that the peptide is presented on the carrier surface with a specific conformation, which is maintained and stabilized by restricting said antigenic peptide in its three dimensional freedom of movement so that conformational changes are prevented or severely restricted.

When a vesicle, a particle or a particulate body is used as a carrier/adjuvant such as, for example, a liposome, the composition of the antigenic peptide may be chosen such that its overall net charge is identical to that of the carrier/adjuvant surface to which the peptide is attached. Electrostatic repulsion forces being effective between the identically charged carrier/adjuvant surface and the antigenic peptide, but particularly the identically charged carrier surface and the amino acid residues constituting the antigenic peptide and more particularly the identically charged carrier surface and the identically charged amino acid residues comprised in the antigenic peptide, may lead to the antigenic peptide taking on a defined, highly specific and stabilized conformation which guarantees a high biological activity. As a result, the antigenic peptide is exposed and presented in a conformation that is highly biologically active in that it allows the immune system of the target organism to freely interact with the antigenic determinants contained in the antigenic construct in the biologically active conformation, which leads to a strong and conformation-specific immune response, resulting in, for example, a high antibody titer in the target organism.

The immunogenic response may be further increased by using a liposome as a carrier, which liposome may function as an adjuvant to increase or stimulate the immune response within the target animal or human to be treated with the therapeutic vaccine according to the invention. Optionally, the liposome may, in addition, contain a further adjuvant such as, for example, lipid A, alum, calcium phosphate, interleukin 1, and/or microcapsules of polysaccharides and proteins, but particularly a detoxified lipid A, such as monophosphoryl or diphosphoryl lipid A, or any other adjuvant that can be suitably used within the scope of the present invention such as, for example, alum, calcium phosphate, interleukin 1, and/or microcapsules of polysaccharides and proteins, LPS, CpG ODN, Pam2CSK4, Pam3CSK4, dsRNA, ssRNA, muramyl dipeptide, Quil Q, QS-21.

Liposomes that can be used in the herein described compositions include those known to one skilled in the art. Any of the standard lipids useful for making liposomes may be used. Standard bilayer and multi-layer liposomes may be used to make compositions as described herein. While any method of making liposomes known to one skilled in the art may be used, the most preferred liposomes are made according to the method of Alving et al., Infect. Immun. 60:2438-2444, 1992, (Lauer *et al.,* 1992) The liposome may have a dual function in that it can be used as a carrier comprising the supramolecular construct as described herein before and, at the same time, function as an adjuvant to increase or stimulate the immune response within the target animal or human to be treated with the herein described therapeutic vaccine.

Optionally, the liposome may, in addition, contain a further adjuvant or and immunomodulator or both such as, for example, lipid A, alum, calcium phosphate, interleukin 1, and/or microcapsules of polysaccharides and proteins, but particularly a lipid A, more particularly a detoxified lipid A, such as monophosphoryl or diphosphoryl lipid A, or alum.

The liposome may be composed of constituents selected from the group consisting of dimyristoyl phosphatidyl choline (DMPC), dimyristoyl phosphatidyl ethanolamine (DMPEA), dimyristoyl phosphatidyl glycerol (DMPG) and cholesterol.

It is further contemplated herein using as a replacement for cationic lipids in the liposomal membrane, anionic lipids selected from the group consisting of:
a. diacyl-phospholipids with headgroups phosphatidyl glycerol, phosphatidyl serine, phosphatidyl inositol, L-α-phosphatidylinositol-4-phosphate or phosphatidic acid;
b. lyso-phospholipids with headgroups phosphatidyl glycerol, phosphatidyl serine or phosphatidic acid, and
c. cardiolipin, dilyso-cardiolipin, monolyso-cardiolipln.

In one aspect, it is contemplated using as a replacement for anionic lipids in the liposomal membrane, cationic lipids selected from the group consisting of:
a. diacyl-phospholipids with headgroups 3-trimethylammonium-propane ,3-dimethylammonium-propane, 3-ethylphosphocholine or 3-phosphatidylethanolamine; and
b. D-erythro-sphingosine, dimethyldioctadecylammonium bromide, N-[1-(2,3-dimyristyloxy)propyl]-N,N-dimethyl-N-(2-hydroxyethyl) ammonium bromide, N,N,N-trimethyl-2-bis[(1-oxo-9-octedecenyl)oxy]-(Z,Z)-1-propanaminium methyl sulfate or 3β-[N-(N',N'-dimethylaminoethane)-carbamoyl]cholesterol hydrochloride.

In particular, the lipid chains attached to the above headgroups can
a. be saturated or unsaturated,
b. vary in length from (CH₂)*ₙ* wherein *n* is between 3 and 24, and
be symmetrically or asymmetrically substituted.

The antigenic composition as described herein may comprise a liposome preparation and an adjuvant, particularly lipid A, alum, calcium phosphate, interleukin 1, and/or microcapsules of polysaccharides and proteins, but particularly a lipid A, more particularly a detoxified lipid A, such as monophosphoryl or diphosphoryl lipid A, or alum.

The herein described liposomal composition comprising an Aβ peptide coupled with lipophilic moieties embedded into liposomes as described herein may be prepared according to the methodology disclosed herein.

When liposomes are used as a carrier/adjuvant, the antigenic peptide as described herein may further be modified by coupling to a lipophilic or hydrophobic moiety that facilitates insertion into the lipid bilayer of the liposome carrier/adjuvant. Said hydrophobic moiety may be a fatty acid, a triglyceride, a diglyceride, a steroid, a sphingolipid, a glycolipid or a phospholipid.

In particular, the lipophilic or hydrophobic moiety may be an alkyl group or a fatty acid with a carbon backbone of at least 1 carbon atom, particularly of at least 2 carbon atoms, particularly of at least 3 carbon atoms, particularly of at least 4 carbon atoms, particularly of at least 6 carbon atoms, particularly of at least 8 carbon atoms, particularly of at least 12 carbon atoms, particularly of at least 16 carbon atoms.

The lipophilic or hydrophobic moieties may be fatty acids, triglycerides and phospholipids, wherein the fatty acid carbon back bone has at least 4 carbon atoms particularly lipophilic moieties having fatty acids with a carbon backbone of at least approximately 14 carbon atoms and up to approximately 24 carbon atoms, more particularly hydrophobic moieties having a carbon backbone of at least 14 carbon atoms. Examples of hydrophobic moieties include, but are not limited to, palmitic acid, stearic acid, myristic acid, lauric acid, oleic acid, linoleic acid, linolenic acid and cholesterol or DSPE. Particularly, the hydrophobic moiety is palmitic acid.

In particular, the antigenic composition as described herein comprises a peptide antigen comprising two palmitic acid moieties, particularly four palmitic acid moieties.

Palmitoylation, while providing an anchor for the peptide in the liposome bilayer, due to the relative reduced length of the C_{16:0} fatty acid moiety leads to the peptide being presented exposed on or in close proximity to the liposome surface. Therefore, the cells processing the antigen will have to take up the entire liposome with the peptide.

The antigenic constructs of the present invention may comprise peptides modified via pegylation (using polyethylene glycol (PEG) or modified polyethylene glycol), or modified via other methods such by palmitic acid as described herein before, poly-amino acids (eg poly-glycine, polyhistidine), poly-saccharides (eg polygalacturonic acid, polylactic acid, polyglycolide, chitin, chitosan), synthetic polymers (polyamides, polyurethanes, polyesters) or copolymers (eg. poly(methacrylic acid) and N-(2-hydroxy) propyl methacrylamide) and the like.

If PEG is used in the preparation of the antigenic construct, the free PEG terminus may be covalently attached to a molecule of phosphatidylethanolamine (where the fatty acid can be: myristic, palmitic, stearic, oleic etc. or combination thereof). This supramolecular structure may be reconstituted in liposomes consisting of phospholipids and cholesterol (phosphatidylethanol amine, phosphatidyl glycerol, cholesterol in varied molar ratios). Other phospholipids can be used. Lipid A may be used at a concentration of approximately 40 µg/pmole of phospholipids. In particular, the herein described antigenic constructs may comprise an antigenic peptide sequence as described herein before, covalently attached to pegylated lysine at least one at each terminus, but particularly 1 or 2 at each terminus. The length of the PEG (polyethylenglycol) chain may vary from n = 8 to n = 150.000 or more, particularly from n = 10 to n = 80.000, more particularly from n = 10 to n = 10.000. In a specific embodiment of the invention the length of the PEG chain is not more than n = 45, particularly between n = 5 and n = 40, more particularly between n = 10 and n = 30, and even more particularly n = 10.

In particular, the Aβ peptide antigen is modified by two palmitic acids bound to the N- and/or C-terminus of the peptide molecule.

Also contemplated herein is post-insertion of different peptide (e.g. antigen) types and/or adjuvant types to the external layer of preformed liposomes in different concentrations as described in EP application 2632434. This post-insertion method comprises pre-forming of liposomes in solution and modification of antigenic peptides through hydrophobic moieties such that the modified antigenic peptide is available in a micellar form. The method further comprises releasing of the antigenic peptides from the micelles by inducing micellar breakdown followed by integration into the pre-formed liposome. This integration process is driven by hydrophobic interactions of the modified antigen and/or the adjuvant with the (phospho)lipid bilayer of the liposomes. In particular, the solubilizing of the modified antigenic peptide and/or adjuvant into the external layer of liposomes is accomplished without the aid of any chemical reaction or additional molecule modification, by diluting the solubilized antigenic peptide or adjuvant (initially presented in micellar form), below the critical micellar concentration of the surfactant. The free form of the antigenic peptide or adjuvant is then integrated in the external layer of the liposomes due to the solubilization of their hydrophobic domains in the acyl moiety of the phospholipids. Thus, the method provides for a stock of "empty liposomes" being disposable for loading according to the respective needs.

In particular, this post-insertion method for preparing a liposome-based antigenic construct comprising an antigenic peptide of the invention and as described herein modified through hydrophobic moieties reconstituted in a liposome, comprises the steps of i) preparing liposomes in solution; ii) preparing a modified antigenic peptide by adding to the N- and/or C-terminus of the peptide molecule at least one hydrophobic moiety; iii) solubilizing the modified antigenic peptide in the presence of a surfactant; iv) diluting the solubilized peptide and, optionally, an adjuvant below the critical micellar concentration (CMC) of the surfactant; and v) loading the preformed liposomes with the diluted, solubilized antigenic peptide and, optionally, the adjuvant, by adding said antigenic peptide and, optionally, said adjuvant to the preformed liposomal preparation and solubilizing the added peptide and, optionally, the added adjuvant into the external layer of the liposomes, particularly without the aid of any chemical reaction or additional molecule modification.

As used herein, the term "critical micellar concentration", also known as CMC, is defined as the concentration of surfactants above which micelles are spontaneously formed. Upon introduction of surfactants (or any surface active materials) into a system the surfactants will initially partition into the interface, thus reducing the systems free energy by a) lowering the energy of the interface (calculated as area x surface tension) and b) by removing the hydrophobic parts of the surfactant from contacts with water. Subsequently, when the surface coverage by the surfactants increases and the surface free energy (surface tension) decreases and the surfactants start aggregating into micelles, thus again decreasing the system's free energy by decreasing the contact area of hydrophobic parts of the surfactant with water. Upon reaching the CMC, any further addition of surfactants will just increase the number of micelles. (IUPAC. Compendium of Chemical Terminology, 2nd ed. Blackwell Scientific Publications, Oxford (1997)).

Advantageously, this method leads to high yields of peptide and/or adjuvant incorporation with a unique molecular display on the liposome facing the external layer of the liposome bilayer. In particular, at least 75%, 76%, 77%, 78%, 79%, 80%, 81%, 82%, 83%, 84%, 85%, 86%, 87%, 89%, 90%, 91%, 92%, 93%, 94%, 95%, 96%, 97%, 98%, 99% and up to 100% of the reconstituted antigenic peptide is present on the surface of the liposome, inserted into the lipid bilayer through its hydrophobic moieties.

The method further results in liposome preparations which show a homogenous size distribution with a polydispersity index in the range of between 0.4 and 0.6, particularly of 0.45 to 0.55, particularly of 0.5. Further, the method and constructs allow for a high bioavailability of peptide and/or adjuvant for the immune system and, as a consequence, an improved immune response. Adjuvant degradation, e.g. MPLA degradation, is minimized or not present at all and, thus, an increased batch reproducibility is provided. The constructs prepared by the above method are stable, capable of sterile filtration and do not induce side immune responses.

Accordingly, further disclosed herein is an antigenic construct comprising a herein described antigenic peptide for use as described herein reconstituted in a liposome, wherein at least 75%, 76%, 77%, 78%, 79%, 80%, 81%, 82%, 83%, 84%, 85%, 86%, 87%, 89%, 90%, 91%, 92%, 93%, 94%, 95%, 96%, 97%, 98%, 99% and up to 100% of the reconstituted antigenic peptide is present on the surface of the liposome, inserted into the lipid bilayer through its hydrophobic moieties.

In particular, a liposomal preparation is described comprising said antigenic construct, which preparation shows a homogenous size distribution with a polydispersity index in the range of between 0.4 and 0.6, particularly of 0.45 to 0.55, particularly of 0.5.

The antigenic peptide may be presented on the surface of the carrier molecule in a highly repetitive array, particularly a repetitive array comprising at least 10 repetitive antigenic units/carrier molecule, particularly at least 50 repetitive antigenic units/carrier molecule, particularly at least 100 repetitive antigenic units/carrier molecule, particularly at least 200 repetitive antigenic units/carrier molecule, particularly at least 300 repetitive antigenic units/carrier molecule; particularly at least 400 repetitive antigenic units/carrier molecule, particularly at least 500 repetitive antigenic units/carrier molecule.

Further, an antibody may be used in the methods as described herein for the treatment, and/or alleviation and/or prevention of memory and/or cognitive impairments or abnormalities, particularly of AD-like memory and/or cognitive impairments or abnormalities, particularly memory and/or cognitive impairments originating in the hippocampus and/or the prefrontal cortex and/or the entorhinal cortex of the brain, particularly impairments of the recognition memory and/or impairments of the contextual associative memory and/or impairments of the associative learning and/or impairments of the declarative memory for facts and events and/or episodic memory impairments and/or language dysfunction such as aphasia and/or visuospatial impairments, such as misplacement of items and difficulty navigating in unfamiliar and familiar terrain and/or decreased executive functions, such as apathy, disinhibition, social isolation, poor judgment, difficulties with planning and/or poor abstract reasoning and/or personality changes and/or emotional changes such as apathy, agitation and psychosis and/or apraxia and/or impairments of performing learned motor tasks and/or other neurological signs including pyramidal and extrapyramidal findings as well as myoclonus or seizures, in children and young to middle-aged subjects with Down's syndrome, which antibody has been generated in response to any one of the antigenic constructs disclosed herein. This antibody may be a polyclonal antibody, a monoclonal antibody, a humanized antibody, a fully human antibody, a diabody, a camelid antibody or a functional fragment of any of the foregoing antibodies, which fragment has substantially the same biological activity in terms of treatment of memory and/or cognitive impairments or abnormalities as the antibody from which said fragment is derived.

Further, an antibody may be used in the methods as described herein for the treatment, and/or alleviation and/or prevention of memory and/or cognitive impairments or abnormalities, particularly of AD-like memory and/or cognitive impairments or abnormalities, particularly memory and/or cognitive impairments originating in the hippocampus and/or the prefrontal cortex and/or the entorhinal cortex of the brain, particularly impairments of the recognition memory and/or impairments of the contextual associative memory and/or impairments of the associative learning and/or impairments of the declarative memory for facts and events and/or episodic memory impairments and/or language dysfunction such as aphasia and/or visuospatial impairments, such as misplacement of items and difficulty navigating in unfamiliar and familiar terrain and/or decreased executive functions, such as apathy, disinhibition, social isolation, poor judgment, difficulties with planning and/or poor abstract reasoning and/or personality changes and/or emotional changes such as apathy, agitation and psychosis and/or apraxia and/or impairments of performing learned motor tasks and/or other neurological signs including pyramidal and extrapyramidal findings as well as myoclonus or seizures, in children with Down's syndrome, which antibody has been generated in response to any one of the antigenic constructs disclosed herein. This antibody may be a polyclonal antibody, a monoclonal antibody, a humanized antibody, a fully human antibody, a diabody, a camelid antibody or a functional fragment of any of the foregoing antibodies, which fragment has substantially the same biological activity in terms of treatment of memory and/or cognitive impairments or abnormalities as the antibody from which said fragment is derived.

In the alternative, an antibody may be used in the methods as described herein for the treatment, and/or alleviation and/or prevention of memory and/or cognitive impairments or abnormalities, particularly of AD-like memory and/or cognitive impairments or abnormalities, particularly memory and/or cognitive impairments originating in the hippocampus of the brain, particularly impairments of the recognition memory and/or impairments of the contextual associative memory and/or impairments of the associative learning and/or impairments of the declarative memory for facts and events and/or episodic memory impairments and/or language dysfunction such as aphasia and/or visuospatial impairments, such as misplacement of items and difficulty navigating in unfamiliar and familiar terrain and/or decreased executive functions, such as apathy, disinhibition, social isolation, poor judgment, difficulties with planning and/or poor abstract reasoning and/or personality changes and/or emotional changes such as apathy, agitation and psychosis and/or apraxia and/or impairments of performing learned motor tasks and/or other neurological signs including pyramidal and extrapyramidal findings as well as myoclonus or seizures, in children subjects with Down's syndrome, which antibody has been generated in response to any one of the antigenic constructs disclosed herein. This antibody may be a polyclonal antibody, a monoclonal antibody, a humanized antibody, a fully human antibody, a diabody, a camelid antibody or a functional fragment of any of the foregoing antibodies, which fragment has substantially the same biological activity in terms of treatment of memory and/or cognitive impairments or abnormalities as the antibody from which said fragment is derived.

In particular, the antigenic peptide fragment derived from amyloid protein or amyloid-like protein may be used in a method as disclosed herein for the treatment and/or alleviating and/or prevention of memory and/or cognitive impairments originating in the hippocampus and/or the prefrontal cortex and/or the entorhinal cortex of the brain.

Further disclosed herein is a method for treating and/or alleviating and/or preventing memory impairments or abnormalities in a subject with Down's syndrome comprising administering to said subject an antigenic peptide or a composition as described herein.

Also disclosed is a method for treating and/or alleviating and/ or preventing cognitive impairments or abnormalities in a subject with Down's syndrome comprising administering to said subject an antigenic peptide or a composition as described herein.

In particular said impairment or abnormality concerns the recognition memory.

In particular, said antigenic peptide for use in a method of enhancing or restoring recognition memory in subjects with Down's syndrome is derived from the N-terminal part of the Aβ peptide, particularly said antigenic peptide consists of all or part of amino acid residues Aβ1-15, Aβ1-16, Aβ1-17, Aβ1-18, Aβ1-19, Aβ1-20, Aβ1-22, or Aβ1-23.

In particular said antigenic peptide consists of amino acid residues Aβ1-15.

Said antigenic peptide for use in a method of enhancing or restoring recognition memory in subjects with Down's syndrome may be derived from the N-terminal part of the Aβ peptide, particularly said antigenic peptide consists of all or part of amino acid residues.

In particular said Impairment or abnormality concerns the contextual associative memory.

In the alternative said antigenic peptide for use in a method of enhancing or restoring contextual associative memory in subjects with Down's syndrome may be derived from the N-terminal part of the Aβ peptide, particularly said antigenic peptide consists of all or part of amino acid residues Aβ1-15, Aβ1-16, Aβ1-17, Aβ1-18, Aβ1-19, Aβ1-20, Aβ1-22, or Aβ1-23.

In particular said antigenic peptide consists of amino acid residues Aβ1-15.

In particular said impairment or abnormality concerns the associative learning.

Said antigenic peptide for use in a method of enhancing or restoring associative learning in subjects with Down's syndrome may be derived from the N-terminal part of the Aβ peptide, particularly said antigenic peptide consists of all or part of amino acid residues Aβ1-15, Aβ1-16, Aβ1-17, Aβ1-18, Aβ1-19, Aβ1-20, Aβ1-22, or Aβ1-23.

Further disclosed herein is an active agent, which is an immunotherapy agent. Non-limiting examples of immunotherapy agents, include inflammatory agents, biological factors, immune regulatory proteins, human and humanized antibodies, and immunotherapy drugs, such as AZT and other derivatized or modified nucleotides. Small molecules can also be employed as agents in the present invention.

In particular said antigenic peptide consists of amino acid residues Aβ1-15.

Further described herein is an antigenic peptide for use in a method of enhancing or restoring associative learning in subjects with Down's syndrome derived from the C-terminal part of the Aβ peptide, particularly said antigenic peptide consists of all or part of amino acid residues Aβ20-36, Aβ20-40, Aβ20-42, Aβ21-36, Aβ21-40, Aβ21-42, Aβ22-36, Aβ22-40 or Aβ22-42.

In particular said antigenic peptide consists of amino acid residues Aβ22-35.

Antigenic peptides which are particularly useful for enhancing and/or restoring associative learning in subjects with Down's syndrome such as, for example antigenic peptide consisting of amino acid residues Aβ22-35, may be combined with one or more biologically active compounds, which have a positive effect on recognition memory and/or contextual associative memory.

In particular, said subject with Down's Syndrome, is a young to middle-aged subject.

In the alternative, said subject is a young-aged subject; or
said subject is a children-aged subject.

In particular, said subjects are below age 60, particularly below age 55, particularly below age 50, particularly below age 45, particularly below 40, particularly below 35, particularly below 30, particularly below 25, particularly below 20, particularly below 15, particularly below age 10, particularly below age 5, particularly below age 3.

The terms "antibody" or "antibodies" as used herein is an art recognized term and is understood to refer to molecules or active fragments of molecules that bind to known antigens, particularly to immunoglobulin molecules and to immunologically active portions of immunoglobulin molecules, i.e molecules that contain a binding site that immunospecifically binds an antigen. The immunoglobulin according to the invention can be of any type (IgG, IgM, IgD, IgE, IgA and IgY) or class (IgG1, IgG2, IgG3, IgG4, IgA1 and IgA2) or subclasses of immunoglobulin molecule.

"Antibodies" are intended within the scope of the present invention to include monoclonal antibodies, polyclonal, chimeric, single chain, bispecific, simianized, human and humanized antibodies as well as active fragments thereof. Examples of active fragments of molecules that bind to antigens include Fab and F(ab')₂ fragments, including the products of an Fab immunoglobulin expression library and epitope-binding fragments of any of the antibodies and fragments mentioned above.

These active fragments can be derived from an antibody as described herein by a number of techniques. For example, purified monoclonal antibodies can be cleaved with an enzyme, such as pepsin, and subjected to HPLC gel filtration. The appropriate fraction containing Fab fragments can then be collected and concentrated by membrane filtration and the like. For further description of general techniques for the isolation of active fragments of antibodies, see for example, Khaw, B. A. et al. J. Nucl. Med. 23:1011-1019 (1982); Rousseaux et al. Methods Enzymology, 121:663-69, Academic Press, 1986.

A "humanized antibody" refers to a type of engineered antibody having its CDRs derived from a non-human donor immunoglobulin, the remaining immunoglobulin-derived parts of the molecule being derived from one (or more) human immunoglobulin(s). In addition, framework support residues may be altered to preserve binding affinity. Methods to obtain "humanized antibodies" are well known to those skilled in the art. (see, e.g., Queen et al., Proc. Natl Acad Sci USA, 86:10029-10032 (1989), Hodgson et al., Bio/Technoloy, 9:421 (1991)).

A "humanized antibody" may also be obtained by a novel genetic engineering approach that enables production of affinity-matured humanlike polyclonal antibodies in large animals such as, for example, rabbits.

The term "monoclonal antibody" is also well recognized in the art and refers to an antibody that is mass produced in the laboratory from a single clone and that recognizes only one antigen. Monoclonal antibodies are typically made by fusing a normally short-lived, antibody-producing B cell to a fast-growing cell, such as a cancer cell (sometimes referred to as an "immortal" cell). The resulting hybrid cell, or hybridoma, multiplies rapidly, creating a clone that produces large quantities of the antibody.

The term "memory and/or cognitive impairments and abnormalities" refers mainly to clinical symptoms associated with amyloid-related pathology in subjects with Down's syndrome (DS), but particularly to impairments and abnormalities originating in the hippocampus, the prefrontal cortex and/or the entorhinal cortex of the brain. Examples of such memory and/or cognitive impairments are i.e., but without being limited thereto, impairments of recognition memory and/or impairments of the contextual associative memory and/or impairments of the associative learning and/or impairments of the declarative memory for facts and events and/or episodic memory impairments and/or language dysfunction such as aphasia and/or visuospatial impairments, such as misplacement of items and difficulty navigating in unfamiliar and familiar terrain and/or decreased executive functions, such as apathy, disinhibition, social isolation, poor judgment, difficulties with planning and/or poor abstract reasoning and/or personality changes and/or emotional changes such as apathy, agitation and psychosis and/or apraxia and/or impairments of performing learned motor tasks and/or other neurological signs including pyramidal and extrapyramidal findings as well as myoclonus or seizures.

The term "memory impairment" further refers to the cardinal feature of DS and is often its earliest manifestation. Even when not the primary complaint, memory deficits can be elicited in most patients with DS at the time of presentation. The pattern of memory impairment in DS is quite distinctive. Declarative memory for facts and events, which depends on medial temporal structures such as the entorhinal cortex and hippocampus, is profoundly affected in DS, while subcortical systems supporting procedural memory are relatively spared until quite late in the course of the disease.

Episodic memory is more profoundly impaired in young DS patients, compared with memory for facts such as vocabulary and concepts (semantic memory), which often becomes impaired somewhat later. Within episodic memory, there is a distinction between immediate recall (eg, mental rehearsal of a phone number), memory for recent events (which comes into play once material that has departed from consciousness must be recalled), and memory of more remote events. Memory for recent events, served by the hippocampus, entorhinal cortex in the medial temporal lobe, is prominently impaired in young DS patients. In contrast, immediate memory (encoded in the prefrontal cortices) is spared early on, as are memories that are consolidated for long periods of time (years), which can be recalled in the absence of hippocampal functioning.

Impairments of procedural memory appear only in middle-aged DS patients. Dysfunction in language, executive function and other cognitive domains develop at variable rates of over the course of the disease. The heterogeneity of the clinical presentation of DS presumably reflects a variable topographical distribution of the burden of brain pathology. Language dysfunction, that is aphasia, is a common symptom of DS, and the first manifestations of language dysfunction usually include word-finding difficulties, circumlocution, and reduced vocabulary in spontaneous speech and with anomia on confrontational naming tests. This can progress to include paraphasic errors, impoverished speech content, and impaired comprehension. However, patients can usually repeat phrases verbatim until the disease is quite advanced. The language difficulties in DS have often been described as Wernicke's and Broca's type of aphasia. When asked to generate word lists in one minute's time, patients with DS perform significantly worse on a category fluency test (eg, lists of animals) than on a letter fluency test (eg, lists of words beginning with F). This reflects the specific deficit in semantic memory.

Loss of visuospatial skills is another early feature of DS that can sometimes be quite prominent at presentation. Visuospatial impairments manifest as misplacement of items and difficulty navigating in first unfamiliar then familiar terrain. Visual agnosia (inability to recognize objects) and prosopagnosia (inability to recognize faces) are later features.

Impairment in executive function is also seen in patients with DS. These symptoms include poor insight, and a reduced ability for abstract reasoning. As the disease progresses, personality changes (such as apathy, social disengagement, and disinhibition), poor judgment and planning, and an inability to complete tasks typically emerges. Superimposed depression, which can be difficult to diagnose in the setting of dementia can also present in this manner.

Reduced insight into deficits (anosognosia) is another characteristic feature of DS. It is not uncommon for patients to deny or underestimate their deficits and offer explanations for them when they are pointed out. Interviewing a collateral historian, such as a spouse is critical to obtain an accurate history. In fact, it is often the family member who brings the complaint of cognitive impairment to medical attention. Loss of insight increases over time along with overall disease severity, and can be associated with behavioral disturbances; those with relatively preserved insight are more likely to be depressed, while those with more impaired insight are likely to be agitated, disinhibited, and exhibit psychotic features. The emergence of behavioral disturbances, including agitation, aggression, wandering, and psychosis (hallucinations, delusions, misidentification syndromes) can lead to significant distress for the patient and family members, and is typically seen in later stages of DS.

Younger patients with DS generally have a normal neurologic examination except for the cognitive examination. While pyramidal and extrapyramidal motor signs, myoclonus, and seizures do occur in patients with DS, these are typically late-stage findings . Similarly, frontal release signs (grasp, snout reflexes, gegenhalten) and incontinence are late, rather than early, features of DS.

The herein described immunogenic composition, which may be a therapeutic vaccine, comprises the antigenic construct
as described herein before in a therapeutically or prophylactically effective amount and may be prepared as a liquid solution, or as an injectable suspension, or else in a solid form suitable for solubilization prior to injection in the context of, for example, a kit for making use of the present composition, as described below.

A "therapeutically or prophylactically effective amount" refers to the amount of antibody, peptide, compound or pharmaceutical composition which, when administered to a human or animal, leads to a therapeutic or prophylactic effect in said human or animal. The effective amount is readily determined by one of skill in the art following routine procedures.

The herein described immunogenic composition may be administered to a human or animal, particularly to a human or animal with Down's syndrome suffering from AD-like cognitive impairments or abnormalities, particularly impairments or abnormalities originating in the hippocampus and/or the prefrontal cortex and/or the entorhinal cortex of the brain, to induce an immune response in said human or animal to alleviate said AD-like symptoms associated with the disease or to restore a condition found in healthy individuals which are unaffected by the disease.

Since virtually all Down's Syndrome people will eventually suffer from AD-like cognitive impairments at some point in life, it will also be beneficial to administer the said vaccine to Down's Syndrome people before the manifestation of AD-like impairments. The said vaccine would then act as a preventive treatment.

The herein described immunogenic composition may be administered to a human or animal by any appropriate standard routes of administration. In general, the composition may be administered by topical, oral, rectal, nasal or parenteral (for example, intravenous, subcutaneous, or intramuscular) routes. In addition, the composition may be incorporated into sustained release matrices such as biodegradable polymers, the polymers being implanted in the vicinity of where delivery is desired, for example, at the site of a tumor. The method includes administration of a single dose, administration of repeated doses at predetermined time intervals, and sustained administration for a predetermined period of time.

The herein described antigenic construct, particularly an immunogenic composition comprising said antigenic construct in a therapeutically effective amount, is administered in repeated doses, in particular in 1 to 15 doses, more particularly in 2 to 10 doses, more particularly in 3 to 7 doses and even more particularly in 4 to 6 doses, in time intervals of between 1 and 10 weeks, particularly in time intervals of between 1 and 6 weeks, more particularly in time intervals of between 1 and 4 weeks, and even more particularly in time intervals of between 2 and 3 weeks. The immune response is monitored by taking sera samples at a suitable time after boosting, particularly 3 to 10 days after boosting, more particularly 4 to 8 days after boosting and more particularly 5 to 6 days after boosting and determining the immunogenicity of the antigenic construct using known methodology, particularly one of the commonly used immunoassays such as, for example, an ELISA assay.

In particular, the herein described antigenic peptide composition is administered by parenteral, particularly by intra-peritoneal, intravenous, subcutaneous and intra-muscular injection.

The dosage of the composition will depend on the condition being treated, the particular composition used, and other clinical factors such as weight, size and condition of the patient, body surface area, the particular compound or composition to be administered, other drugs being administered concurrently, and the route of administration.

The herein described immunogenic composition may be administered in combination with other biologically active substances and procedures for the treatment of symptoms associated with Down's syndrome. The other biologically active substances may be part of the same composition already comprising the immunogenic composition as described herein, in form of a mixture, wherein the immunogenic composition and the other biologically active substance are intermixed in or with the same pharmaceutically acceptable solvent and/or carrier or may be provided separately as part of a separate compositions, which may be offered separately or together in form of a kit of parts.

The herein described immunogenic composition may be administered concomitantly with the other biologically active substance or substances, intermittently or sequentially. For example, the herein described immunogenic composition may be administered simultaneously with a first additional biologically active substance or sequentially after or before administration of said composition. If an application scheme is chosen where more than one additional biologically active substance are administered together with the at least one of the herein described immunogenic compositions, the compounds or substances may partially be administered simultaneously, partially sequentially in various combinations.

Further disclosed herein are thus mixtures of an immunogenic composition as described herein and, optionally, one or more further biologically active substances in a therapeutically or prophylactically effective amount, as well as to methods of using such a composition as described herein, or mixtures thereof for the prevention and/or therapeutic treatment and/or alleviation of the effects of amyloid related pathology in children, or in young to middle aged subjects with Down's syndrome, particularly for amelioration or restoration of memory impairments or abnormalities, particularly impairments and abnormalities originating in the hippocampus and/or the prefrontal cortex and/or the entorhinal cortex of the brain, particularly for amelioration or restoration of the impairments of recognition memory and/or impairments of the contextual associative memory and/or impairments of the associative learning and/or impairments of the declarative memory for facts and events and/or episodic memory impairments and/or language dysfunction such as aphasia and/or visuospatial impairments, such as misplacement of items and difficulty navigating in unfamiliar and familiar terrain and/or decreased executive functions, such as apathy, disinhibition, social isolation, poor judgment, difficulties with planning and/or poor abstract reasoning and/or personality changes and/or emotional changes such as apathy, agitation and psychosis and/or apraxia and/or impairments of performing learned motor tasks and/or other neurological signs including pyramidal and extrapyramidal findings as well as myoclonus or seizures.

The herein described mixtures may comprise, in addition to an immunogenic composition as described herein, a biologically active substance such as, for example, known compounds used in the medication of AD-like symptoms in children, or in young to middle aged subjects with Down's syndrome.

The other biologically active substance or compound may exert its biological effect by the same or a similar mechanism as the herein described immunogenic composition or by an unrelated mechanism of action or by a multiplicity of related and/or unrelated mechanisms of action.

Generally, the other biologically active compound may include antibodies raised against and binding to an antigenic peptide as disclosed herein or compounds used in the medication of neurological disorders such as neutron-transmission enhancers, psychotherapeutic drugs, acetylcholine esterase inhibitors, calcium-channel blockers, biogenic amines, benzodiazepine tranquillizers, acetylcholine synthesis, storage or release enhancers, acetylcholine postsynaptic receptor agonists, monoamine oxidase-A or -B inhibitors, N-methyl-D-aspartate glutamate receptor antagonists, non-steroidal anti-inflammatory drugs, antioxidants, and serotonergic receptor antagonists.

In particular, the mixture as described herein may comprise at least one other biologically active compound selected from the group consisting of compounds against oxidative stress, anti-apoptotic compounds, metal chelators, inhibitors of DNA repair such as pirenzepin and metabolites, 3-amino-1-propanesulfonic acid (3APS), 1,3-propanedisulfonate (1,3PDS), secretase activators, β- and γ-secretase inhibitors, β- and γ-secretase modulators, tau proteins, neurotransmitter, β-sheet breakers, anti-inflammatory molecules, or cholinesterase inhibitors (ChEIs) such as tacrine, rivastigmine, donepezil, and/or galantamine and other drugs and nutritive supplements, together with an therapeutic vaccine according to the invention and, optionally, a pharmaceutically acceptable carrier and/or a diluent and/or an excipient.

The mixtures as described herein may further comprise niacin or mementine together with an immunogenic composition as described herein and, optionally, a pharmaceutically acceptable carrier and/or a diluent and/or an excipient.

Mixtures may comprise "atypical antipsychotics" such as, for example clozapine, ziprasidone, risperidone, aripiprazole or olanzapine for the treatment of positive and negative psychotic symptoms including hallucinations, delusions, thought disorders (manifested by marked incoherence, derailment, tangentiality), and bizarre or disorganized behavior, as well as anhedonia, flattened affect, apathy, and social withdrawal, together with an immunogenic composition and/or a therapeutic vaccine according to the invention and, optionally, a pharmaceutically acceptable carrier and/or a diluent and/or an excipient.

Other compounds that can be suitably used in mixtures in combination with the herein described immunoginic composition are described, for example, in WO 2004/058258 (see especially pages 16 and 17) including therapeutic drug targets (page 36-39), alkanesulfonic acids and alkanolsulfuric acid (pages 39-51), cholinesterase inhibitors (pages 51-56), NMDA receptor antagonists (pages 56-58), estrogens (pages 58-59), non-steroidal anti-inflammatory drugs (pages 60-61), antioxidants (pages 61-62), peroxisome proliferators-activated receptors (PPAR) agonists (pages 63-67), cholesterol-lowering agents (pages 68-75); amyloid inhibitors (pages 75-77), amyloid formation inhibitors (pages 77-78), metal chelators (pages 78-79), anti-psychotics and anti-depressants (pages 80-82), nutritional supplements (pages 83-89) and compounds increasing the availability of biologically active substances in the brain (see pages 89-93) and prodrugs (pages 93 and 94), but especially the compounds mentioned on the pages indicated above.

Further disclosed herein is an antigenic construct which comprises an Aβ peptide that does not contain a T-cell epitope and thus is free of potential side effects such as neurological complications caused by an over-activated complement system. This can be achieved by administering an Aβ peptide antigen, particularly a palmitoylated Aβ peptide antigen, more particularly the palmitoylated Aβ₁₋₁₅ peptide antigen, but especially the palmitoylated Aβ₁₋₁₅ peptide antigen, Aβ₁₋₁₅ in combination with a complement inhibitor.

The complement inhibitor may be a compound selected from the group consisting of soluble human complement Receptor 1, anti- human complement protein C5 such as, for example, a humanized anti C5 monoclonal antibody or a single-chain fragment of a humanized monoclonal antibody, C1-esterase inhibitor-N and Natural human C1 Inhibitor.

The modified amyloid peptide antigen such as, for example, the amyloid beta 1-15 peptide antigen may be synthesized following the method reported in (Nicolau *et al.,* 2002). The approach reported in Nicolau et al may be modified by first synthesizing the antigenic peptide which is then further modified by an on-resin grafting of a lipophilic or hydrophobic moiety, to the terminal amino acid residues of the pre-formed peptide. In particular, a protected amino acid, particularly a Fmoc-protected amino acid, is attached to a resin using known coupling chemistry. The protecting group is removed and a second protected amino acid residue coupled. Then, standard automated peptide synthesis using known protection chemistry, particularly Fmoc/tBu chemistry, and standard side-chain protecting groups are then used to synthesis the Aβ antigenic peptide, particularly the Aβ₁₋₁₅ antigenic peptide by coupling on amino acids 1 to 15 of amyloid protein Aβ₁₋₄₂ to produce the peptide fragment with a given sequence. In a final step two further protected amino acids are coupled to the growing peptide fragment. The Mtt groups on the side chains of first two and last two amino acids can then be selectively cleaved and coupled to palmitic acid. After washing of the resin, the protecting group is removed and the resin simultaneously cleaved, followed by side-chain deprotections using standard methodology. The final product can then be obtained in high purity and its identity confirmed by methods known in the art such as, for example, electrospray mass spectrometry.

The modified amyloid Aβ antigenic peptide, particularly the modified Aβ₁₋₁₅ antigenic peptide may be reconstituted in a construct consisting of liposomes, particularly liposomes made of dimyristoyl phosphatidyl choline (DMPC), dimyristoyl phosphatidyl ethanolamine (DMPEA), dimyristoyl phosphatidyl glycerol (DMPG) and cholesterol, optionally containing monophosphoryl lipid A.

It is also contemplated herein using as a replacement for cationic lipids in the liposomal membrane, anionic lipids selected from the group consisting of:
a. diacyl-phospholipids with headgroups phosphatidyl glycerol, phosphatidyl serine, phosphatidyl inositol, L-α-phosphatidylinositol-4-phosphate or phosphatidic acid;
b. lyso-phospholipids with headgroups phosphatidyl glycerol, phosphatidyl serine or phosphatidic acid, and
c. cardiolipin, dilyso-cardiolipin, monolyso-cardiolipin

In one aspect, it is contemplated using as a replacement for anionic lipids in the liposomal membrane, cationic lipids selected from the group consisting of:
a. diacyl-phospholipids with headgroups 3-trimethylammonium-propane ,3-dimethylammonium-propane, 3-ethylphosphocholine or 3-phosphatidylethanolamine;
b. D-erythro-sphingosine, dimethyldioctadecylammonium bromide, N-[1-(2, 3-dimyristyloxy)propyl]-N,N-dimethyl-N-(2-hydroxyethyl) ammonium bromide, N,N,N-trimethyl-2-bis[(1-oxo-9-octadecenyl)oxy]-(Z,Z)-1-propanaminium methyl sulfate or 3β-[N-(N',N'-dimethylaminoethane)-carbamoyl]cholesterol hydrochloride.

In particular, the lipid chains attached to the above headgroups can
a. be saturated or unsaturated,
b. vary in length from (CH₂)*ₙ* wherein *n* is between 3 and 24, and
be symmetrically or asymmetrically substituted.

Liposomes with lipid A may be used as adjuvant to prepare the anti-amyloid vaccine. Dimyristoylphosphatidyl-choline, - glycerol and cholesterol are mixed, particularly in a molar ratio of 0.9:1.0:0.7. A strong immunmodulator such as, for example, monophosphoryl lipid A is then added at a suitable concentration, particularly at a concentration of between 30 and 50 mg per mmol, more particularly at 40 mg per mmol of phospholipids. The modified antigenic Aβ peptide is then added at a molar ratio peptide to phospholipids of between 1:30 and 1:200, particularly at a molar ratio of between 1:50 and 1:120, more particularly of 1:100. Solvents are removed, for example through evaporation, and the resulting film hydrated with sterile buffer solution such as, for example PBS.

Liposomes may also be prepared by the crossflow injection technique as described, for example, in (Wagner *et al.,* 2002). During the injection of lipid solutions into an aqueous buffer system, lipids tend to form "precipitates", followed by self arrangement in vesicles. The obtained vesicle size depends on factors such as lipid concentration, stirring rate, injection rate, and the choice of lipids. The preparation system may consist of a crossflow injection module, vessels for the polar phase (e.g. a PBS buffer solution), an ethanol/lipid solution vessel and a pressure device, but particularly a nitrogen pressure device. While the aqueous or polar solution is pumped through the crossflow injection module the ethanol/lipid solution is injected into the polar phase with varying pressures applied. Various methods of production of the liposome antigenic constructs are described in WO2007/068411.

Accordingly, the liposomal composition for use in the herein described method may be prepared to comprise a palmitoylated Aβ 1-15, particularly a tetrapalmitoylated Aβ 1-15 together with monophosphoryl lipid A (MPLA) as an adjuvant.

Four to ten, particularly five to six doses of the liposomal composition may be administered subcutaneously and weekly or bi-weekly to a subject to be vaccinated. Plasma probes may be obtained and analyzed for IgG titers periodically.

The effectiveness of the liposomal composition of the invention as an immunogen in children and young to middle aged subjects with Down's syndrome and the therapeutic potential for treating memory deficits in said subjects could be demonstrated in a relevant animal model for Down's syndrome. In particular, Ts65Dn mice were used that are widely accepted as an animal model for DS. Ts65Dn mice exert a triplicate of the murine chromosome 16, which hosts the murine APP gene (Davisson *et al.,* 1993;Netzer *et al.,* 2010). These transgenic mice show 1.5 fold increased murine Aβ (Hunter *et al.,* 2004) and demonstrate behavioral deficits in several memory tasks (Belichenko *et al.,* 2009).

It could be demonstrated within the present invention that the liposomal composition of the invention induced elevated titers of antibodies and that said antibodies remain elevated even 40 days after the last immunization. Thus, the liposomal composition as described herein is capable of inducing a robust immune response and of breaking Aβ self-tolerance in treated subjects with Down's syndrome.

The liposomal composition of the invention and as described herein was further shown to be capable to induce in young to middle aged mice with Down's syndrome, IgG titers as high as in subjects of a control group without Down's syndrome. The liposomal composition as described herein induces increased titers of antibodies of the IgG2a isotype as compared to the control group, whereas antibody titers of the IgG1 and IgG2b isotype are comparable in the treatment and the control group. The titers of IgM class antibodies are lower in the treatment group as compared to the control group. This slightly lower IgM level may be caused by the altered immune system of mice with Down's syndrome. The liposomal composition is thus capable of overcoming the impaired adaptive immune response to Aβ described in DS people (Monsonego *et al.,* 2001).

The liposomal composition of the invention and as described herein is also safe and does not induce unwanted side effects. In particular, treatment with the liposomal composition does not lead to cell activation the brain, such as activation of astrocytes nor microglia.

The liposomal composition of the invention and as described herein was shown to result in a higher discrimination ratio in the animal model indicating that treatment led to a significant improvement of memory in the treated animals.

The liposomal composition as described herein was further shown in a test consisting of training, cued and contextual sessions to result in an enhanced freezing in the animal model reaching levels comparable to that observed in the control animals. This suggests that the immunization was efficient and enhanced the memory capacity of the model animals.

The liposomal composition as described herein is thus capable of rescuing the memory deficits in children and in young to middle aged subjects suffering from Down's syndrome, in particular in children and young to middle-aged subjects with Down's syndrome, who have not yet developed Aβ-associated plaques in the brain. Patients, who suffer from Down's syndrome exhibit cognitive abnormalities such as memory impairment and abnormal activities.

The liposomal composition as described herein is further capable of rescuing the memory deficits in children and in young to middle aged subjects suffering from Down's syndrome, in particular in children and in young to middle-aged subjects with Down's syndrome, who have already developed Aβ-associated plaques in the brain.

In one embodiment, treatment with the liposomal composition can ameliorate or restore the memory deficit in a child or in a young to middle-aged subject with Down's syndrome, particularly impairments of recognition memory and/or impairments of the contextual associative memory and/or impairments of the associative learning and/or impairments of the declarative memory for facts and events and/or episodic memory impairments and/or language dysfunction such as aphasia and/or visuospatial impairments, such as misplacement of items and difficulty navigating in unfamiliar and familiar terrain and/or decreased executive functions, such as apathy, disinhibition, social isolation, poor judgment, difficulties with planning and/or poor abstract reasoning and/or personality changes and/or emotional changes such as apathy, agitation and psychosis and/or apraxia and/or impairments of performing learned motor tasks and/or other neurological signs including pyramidal and extrapyramidal findings as well as myoclonus or seizures.

### BRIEF DESCRIPTION OF DRAWINGS

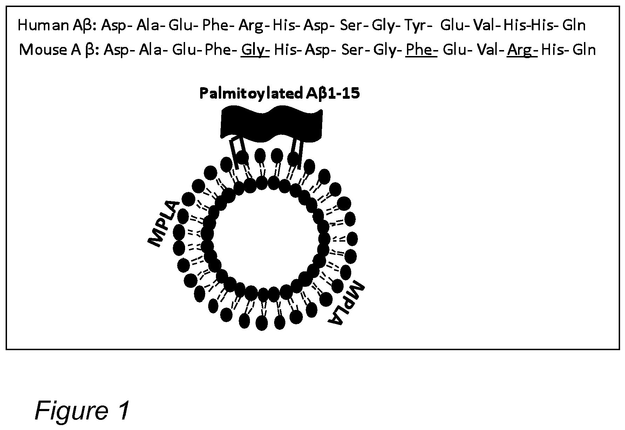

*Figure 1* shows a schematic presentation of the ACI-DS-01 vaccine, which is a liposome-based vaccine with tetra-palmitoylated mouse Aβ1-15 antigen and MPLA as adjuvant. Three amino acid difference between human and mouse Aβ1-15 are underlined.

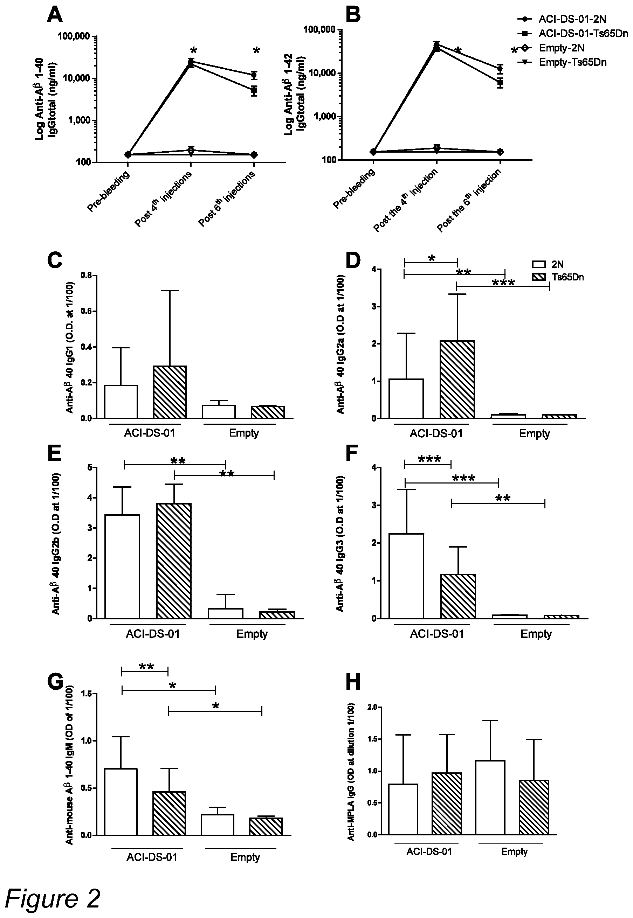

*Figure 2* shows anti-mouse Aβ antibody levels in Ts65Dn mice immunized with the vaccine ACI-DS-01. A and B) Anti-mouse-Aβ40 or 42 IgG titers were detected in the plasma of mice immunized with ACI-DS-01. The induced titers were observed following the second immunization and remained high even 40 days after the 6^{th} injections in comparison to mice immunized with empty-liposome. There was no difference between the measured titers of 2N and Ts65Dn mice. C to F) IgG isotypes were detected following the 4^{th} immunization. G) IgM titers were barely lower in the Ts65Dn mice. H) the same level of anti-MPLA IgG titers were detected in all mice immunized with ACI-DS-01. Graphs represent the mean ± SD. (n=20 2N-ACI-DS-01, n=15 Ts65Dn-ACI-DS-01, n= 18 2N-Empty and n= 11 Ts65Dn-Empty. Graphs represent the mean ± SD).

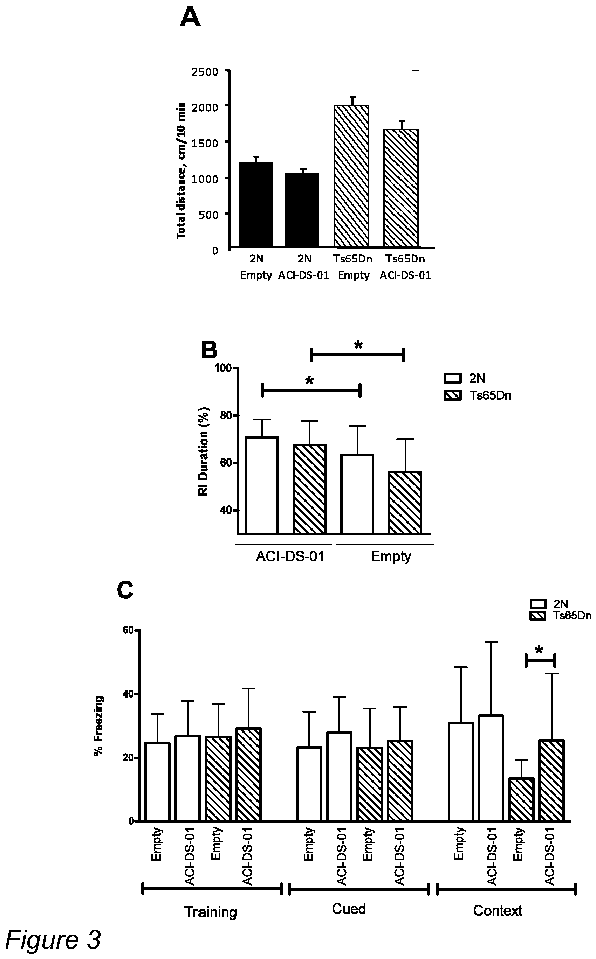

*Figure 3* shows efficacy of immunization on memory performance. The difference in the spontaneous locomotor activity between 2N and Ts65Dn mice remained similar following the immunization. B) Mice immunized with ACI-DS-01 showed a significant enhanced recognition index (RI) in the novel object recognition in comparison to the group treated with Empty vaccine. C) In the fear conditioning, immunized mice showed border line significance for a higher level of freezing during the contextual session. Graphs represent the mean ± SD. (n= 20 2N-ACI-DS-01, n= 13 Ts65Dn ACI-DS-01, n= 18 2N-Empty and n= 11 Ts65Dn-Empty).

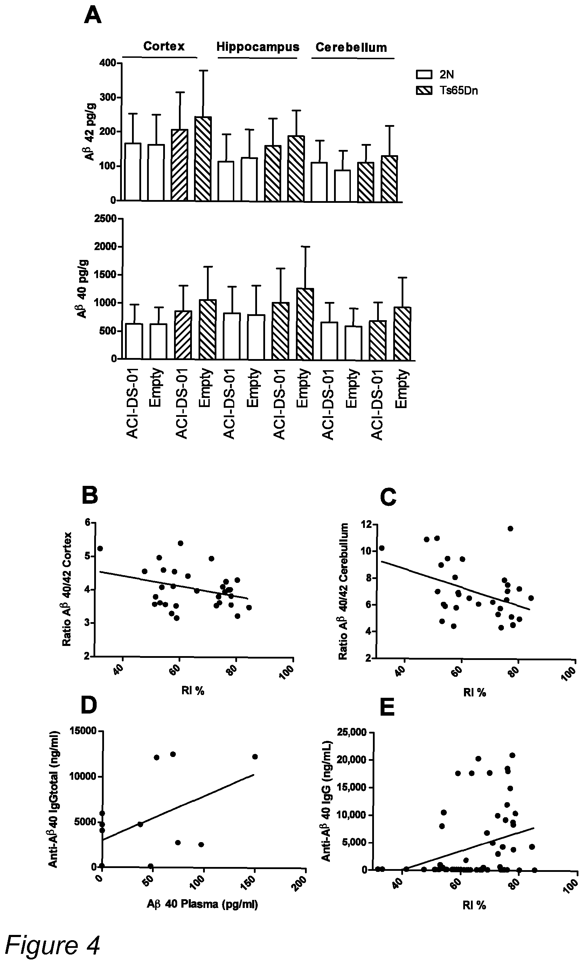

*Figure 4* shows level of Aβ40 in mice treated with ACI-DS-01 vaccine. A) In the cortex, the level of Aβ40 was barely higher in the Ts65Dn in comparison to the 2N mice. The vaccine ACI-DS-01 showed a trend to decrease Aβ42 and Aβ40 levels in the cortex and the hippocampus. Aβ42 and Aβ40 levels in the cerebellum showed a statistical significant decrease following the treatment with ACI-DS-01. Graphs represent the mean ± SD. (n=10 2N-ACI-DS-01, n=10 Ts65Dn ACI-DS-01, n= 8 2N-Empty and n= 5 Ts65Dn-Empty). B and C) Correlation between the ratio Aβ-40/42 in the cortex or the cerebellum with the RI. D) The level of anti-Aβ IgG titers correlate weakly with Aβ40 level in the plasma of Ts65Dn mice treated with the ACI-DS-01. E) Significant correlation between the level of anti-Aβ IgG and the RI.
   (n=19 2N ACI-DS-01, n=13 Ts65Dn ACI-DS-01, n= 18 2N-Empty and n= 11 Ts65Dn-Empty).

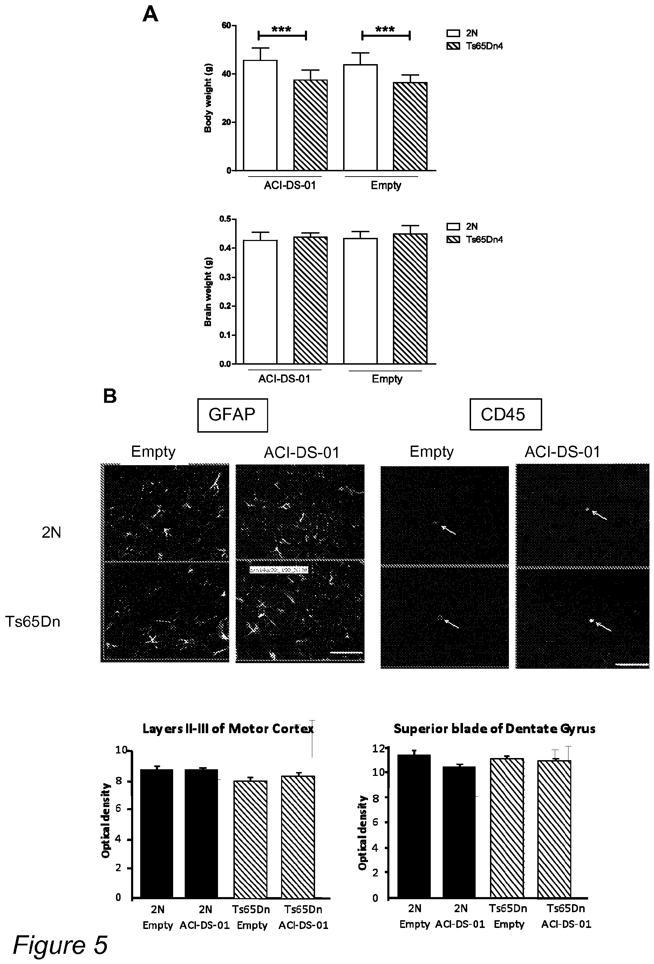

*Figure 5* shows studies of inflammatory reaction. A) Body and brain weight. B) Confocal images of GFAP immunoreactivity (left) and CD45 (right) in treated 2N and Ts65Dn mice. Arrows point individual CD45-positive microglial cells. The optical density of GFAP immunoreactivity was quantified and revealed no difference between groups. n=4 from each group.

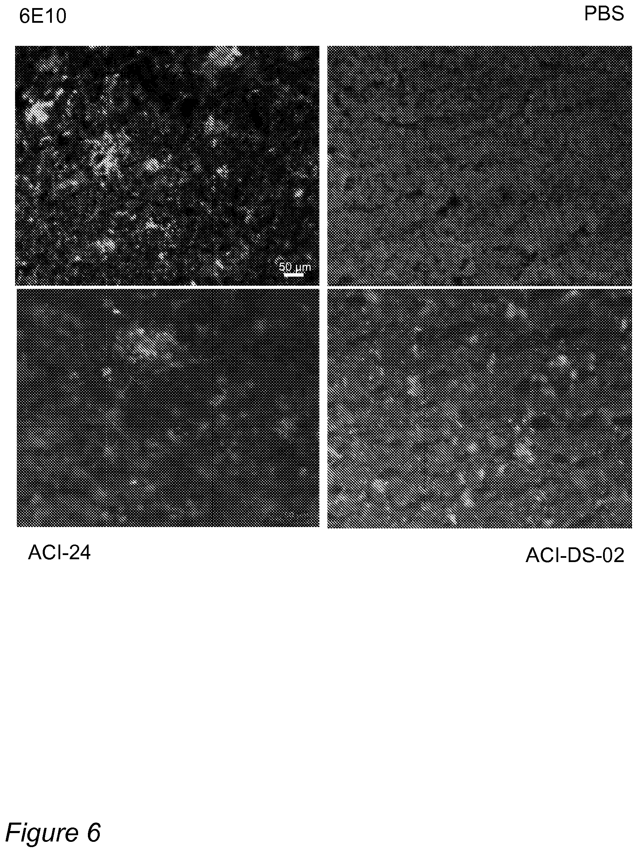

*Figure 6* shows sections of human DS brain samples immuno-stained with sera of mice immunized with ACI-DS-02. Positive area indicates that the ACI-DS-02 derived antibody binds to amyloid plaques in the brain of DS people. The staining was similar to the obtained staining with 6E10, a commercially available anti-amyloid beta antibody, and with ACI-24 derived antibody. ACI-24 vaccine corresponds to ACI-DS-01 vaccine with the difference that the antigen used in ACI-24 is a human Aβ 1-15 sequence, while ACI-DS-01 vaccine contains mouse Aβ 1-15 sequence as the antigen (for three amino acid difference in those two antigens see Figure 1). No staining was observed when a section was incubated with sera of mice immunized with PBS.

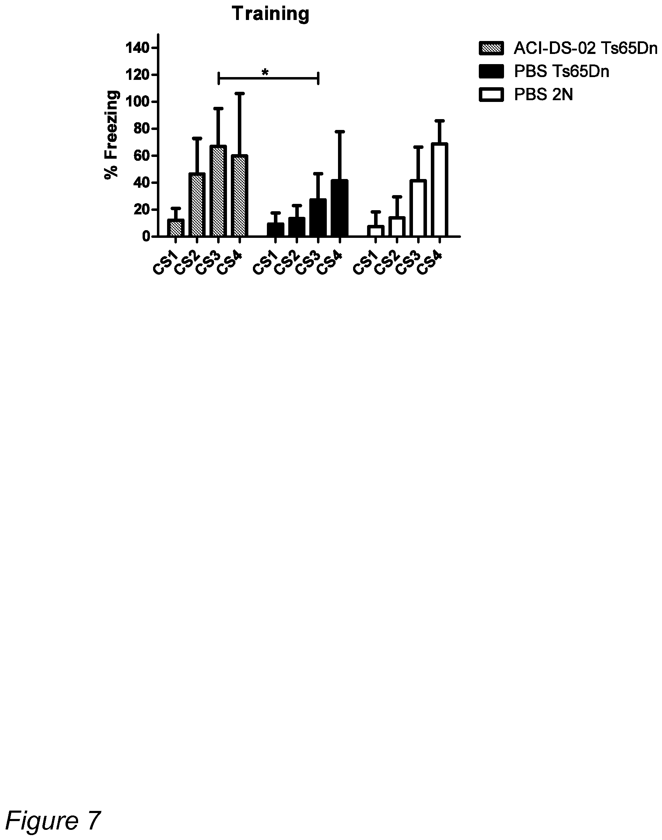

*Figure 7* shows the learning of TS65Dn mice immunized with ACI-DS-02 vaccine in the fear conditioning test. TS65Dn mice immunized with ACI-DS-02 showed greater freezing during the acquisition session in comparison to TS65Dn mice immunized with PBS, particularly following the third conditioned-stimulus (CS).
   Graphs represent the mean ± SD. (n= 7 Ts65Dn-ACI-DS-02, n= 7 2N-PBS and n=4 Ts65Dn-PBS. Graphs represent the mean ± SD).

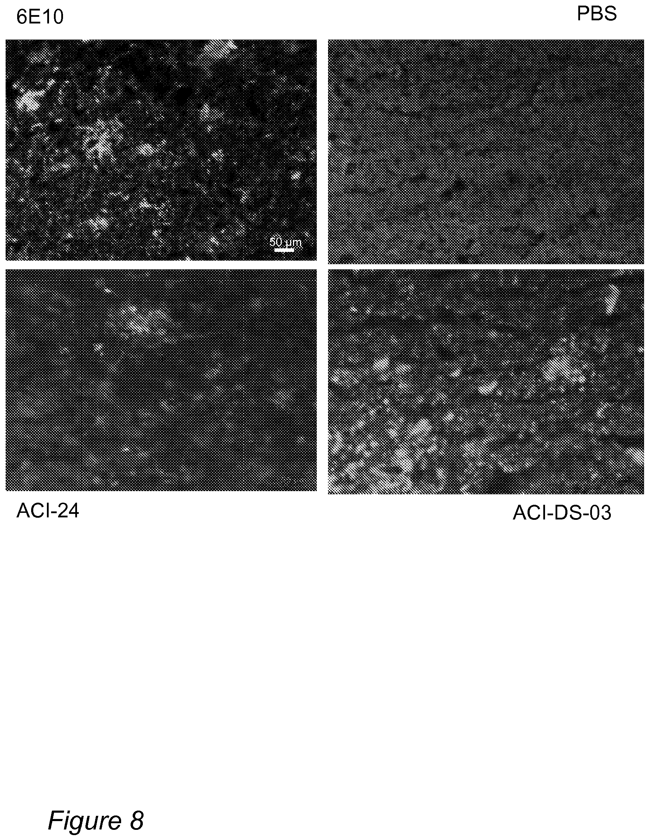

*Figure 8* shows sections of human DS brain samples immuno-stained with sera of mice immunized with ACI-DS-03. Positive area indicates that the ACI-DS-03 derived antibody binds to amyloid plaques in the brain of DS people. The staining was similar to the obtained staining with 6E10, a commercial available anti-amyloid beta antibody, and with ACI-24 derived antibody. ACI-24 vaccine corresponds to ACI-DS-01 vaccine with the difference that the antigen used in ACI-24 is a human Aβ 1-15 sequence, while ACI-DS-01 vaccine contains mouse Aβ 1-15 sequence as the antigen (for three amino acid difference in those two antigens see Figure 1). No staining was observed when section was incubated with sera of mice immunized with PBS.

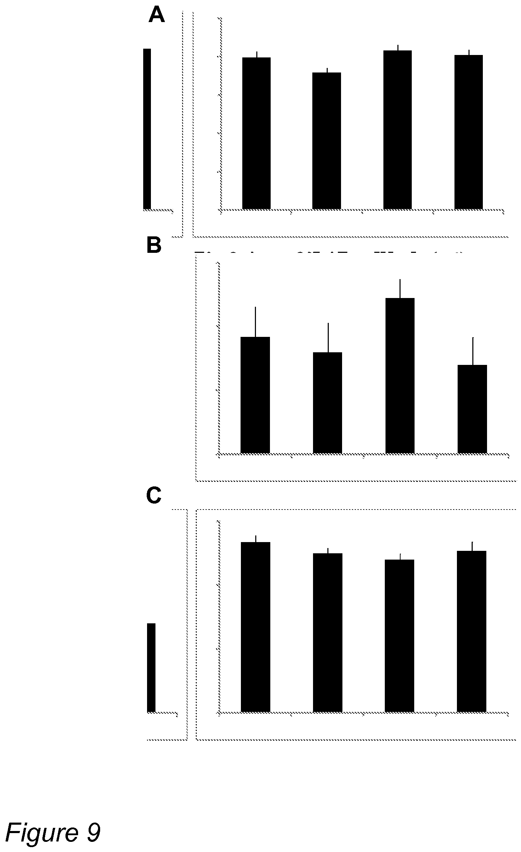

*Figure 9* shows the morphological analysis of Ts65Dn mice immunized with ACI-DS-01 vaccine. A) Number of ChAT+ cells in medial septum and B) optical density and were similar in all groups. C) area of ChAT+ cell body were increased in Ts65Dn mice immunized with ACI-DS-01 in comparison to Ts65Dn treated with Empty vaccine. (n= 4 2N-Empty, n= 4 Ts65Dn-Empty, n= 4 2N-ACI-DS-01 and n= 4 Ts65Dn-ACI-DS-01. Graphs represent the mean ± SD).

### EXAMPLES

### Example 1 General Methodology

### 1.1 Animals:

Ts65Dn mice known as a DS mouse model (Davisson *et al.,* 1993) were used (n =30) and the age matched control 2N (n = 40). At starting date, the used mice were 5 months old. At the end of the study (immunization and behavioral testing), mice were 9 months old. Therefore, all the samples collected at sacrifice are from 9 months old mice.

Ts65Dn newborns have elevated lethality (around 6%) mainly due to congenital heart malformations (Randall 2006, Moore 2006). However, mice can survive even up to 18 to 24 months. In our study, a death rate of 15% was observed. The survived mice are -20% smaller in size compared to normal littermates. The most severely affected mice that died at birth, do not survive to be analyzed, resulting in underestimates of the impact of the trisomic genes on some phenotypes (i.e heart defect). However, for our concerns, the surviving mice represent an adequate model of DS for analyzing amyloid and AD-like pathologies.

In the DS mouse model used for these studies the mice show increased levels of amyloid at the age of 4 months (Hunter 2004). At an age of 9 months, the level of amyloid beta reaches 3 fold the normal level as is predicted in view of the three copies of the APP gene. Similar to DS people, aging is an important factor for amyloid deposition in Ts65Dn brains. Despite the age-related accumulation of amyloid, the TS65Dn mice do not develop plaques. Nevertheless, they do replicate faithfully the degeneration of neuronal populations that are seen in AD and in people with Down syndrome (Salehi et al., 2009)

Taken together, the DS-features in the used mouse-model comprise the presence of pathological proteins and phenotypical aspects; i.e amyloid load and cognitive impairment. Therefore, Ts65Dn mice can accurately recapitulate the pathogenic processes of DS. Since these mice start to accumulate amyloid at a comparable level to humans at 6 months of age, the age of the model mice compares to human DS patients at an age range from young to middle age.

Mice used for immunization study with ACI-DS-01 vaccine were the Ts65Dn mouse colony maintained for more than 10 generations by crossing B6EiC3Sn-Ts(1716)65Dn females (Jackson Laboratory, Bar Harbor, ME) with B6EiC3Sn F1/J A/a males (Jackson Laboratory). This breeding scheme was used because trisomic mice breed very poorly or not at all when inbred; the B6C3 background has been the most successful. To distinguish 2N from Ts65Dn mice, genomic DNA was extracted from tail samples. A quantitative polymerase chain reaction (PCR) protocol (provided by the Jackson Laboratory) was used to measure Mx1 gene expression, which is present in three copies in Ts65Dn. Male mice were used in all studies. They were 4+/-0.3 months at the beginning of the study.

**1.2 Vaccine preparation - Preparation of a liposome-based antigenic construct**

| Vaccine code | Antigenic peptide sequence^{a} | Aβ sequence | Antigenic peptide conformation within the vaccine (%) measured by ATR-IR |
|---|---|---|---|
| ACI-DS-01 | H-Lys(Pal)-Lys(Pal)-Asp-Ala-Glu-Phe-Gly-His-Asp-Ser-Gly-Phe-Glu-Val-Arg-His-Gln-Lys(Pal)-Lys(Pal)-OH | Mouse Aβ1-15^{b} (SEQ ID NO: 2) | 73% beta sheet 19% random coil 0% alpha helix or loops 8% beta turns |
| ACI-DS-02 | H-Lys(Pal)-Glu-Asp-Val-Gly-Ser-Asn-Lys-Gly-Ala-Ile-Ile-Gly-Leu-Met-Lys(Pal)-OH | Human (SEQ ID NO: 4) and mouse (SEQ ID NO: 5) Aβ22-35 | 67% beta sheet 21% random coil 0% alpha helix or loops 12% beta turns |
| ACI-DS-03 | H-Lys(Pal)-Lys(Pal)-His-Gln-Lys-Leu-Val-Phe-Phe-Ala-Glu-Asp-Val-Gly-Ser-Asn-Lys-Gly-Lys(Pal)-Lys(Pal)-OH | Human (SEQ ID NO: 6) and mouse (SEQ ID NO: 7) Aβ14-29 | 57% beta sheet 19% random coil 12% alpha helix or loops 11% beta turns |

| | | | |
|---|---|---|---|
| Peptide sequences are presented in three-letter code; Pal stands for palmitoylated residue.; ^{b}Three amino acid difference between human and mouse Aβ1-15 sequence are underlined and shown in Figure 1. | | | |

The liposome antigenic construct is produced according to the method described in WO2007/068411. Palmitoylated peptides were prepared by PolyPeptide Laboratories (Strasbourg, France). In summary, the liposomal vaccines were prepared by solubilizing dimyristoylphosphatidylcholine (DMPC), dimyristoylphosphatodyl-glycerol (DMPG), cholesterol and monophosphryl Lipid A (MPLA), (all Avanti Polar Lipids, AL, USA), at molar ratios 9:1:7:0.05, respectively, in ethanol at 60°C. The lipid/ethanol solution was diluted in PBS pH 7.4, allowing the formation of multi-layer vesicles. The resulting preparation was then concentrated by ultrafiltration (Vivaflow 200 - 100.000 MWCO Polyethersulphone), with a flow rate of 200 ml/min and the reaction volume was reduced with an ultrafiltration step. The concentrated solution was further submitted to dialyses in a Vivaflow 200 device where a 10x volume exchange was performed with PBS pH 7.4. The multilamelar liposomes were then submitted to homogenization (7 cycles at 15.000-20.000 Psi), followed by sequential 3 extrusion cycles through polycarbonate filters (Whatman) with a pore size of 0.2 µm and a diameter of 47 mm. Both the homogenization and extrusion steps were done in an EmulsiFlex-C5 (Avestin, Canada). The resulting unilamelar liposomes with no antigen were diluted in PBS pH 7.4 and heated to 60°C prior to peptide addition. The palmitoylated peptide was dissolved in PBS pH 11.4 with 5.0% (β-OG). For the preparation of vaccines ACI-DS-01, ACI-DS-02 and ACI-DS-03, the corresponding antigenic peptides were used at 1.33, 0.67 and 1.06 mg/mL respectively. The resulting solution comprised a detergent concentration above its critical micellar concentration (CMC) of 0.73 % (wt/v). This peptide solution was then injected into the liposome solutions at 60°C and stirred for 30 min, resulting in a solution having a final β-OG concentration far below its critical micellar concentration. The detergent micellar breakdown, induces the incorporation of the palmitoylated antigen on the surface of the previously formed liposomes. This liposomal solution with antigen was then concentrated through ultrafiltration and submitted to 10x volume exchange with PBS pH 7.4 by diafiltration (both in a 100.000 MWCO Polyethersulphone with a flow of 200 ml/min). The resulting liposomes were then twice sterile filtered by passing through 0.2 µm polycarbonate syringe filters and stored at 5°C. The peptide to lipid molar ratio in the vaccine was 1:100.

### 1.3 Immunization with ACI-DS-01 vaccine

All mice received subcutaneous (s.c.) immunizations with 200µl of ACI-DS-01 (51.2 µg per dose of Pal1-15, tetrapalmitoylated Aβ peptide 1-15) or empty liposome vaccine. A total of six injections were done at day 1, 14, 28, 42, 56, 70 and 110. On the same days, tail bleedings were performed just before the first vaccine injection on day -1 (pre-bleeding), day 56 (14 days after the 4th injection) and at sacrifice, day 110 (40 days after the 6th injection). Mice were sacrificed at the end of the study at the age of 9 months and brains were collected.

### 1.4 Quantification of mouse Aβ-specific antibodies after ACI-DS-01 immunization

Aβ1-42-specific IgG responses were determined by ELISA. Briefly, plates were coated with 10 µg/mL of mouse Aβ1-42 (Bachem H5966, lot: 1013710, 1028321 or 1033165) or mouse Aβ1-40 (Bachem H5638, lot: 1013645) overnight at 4°C. After washing with PBS-0.05% Tween 20 and blocking with 1% BSA, serial dilutions of plasma were added to the plates and incubated at 37°C for two hours. The antibody anti-Aβ 4G8 (Covance SIG-39220, lot: 08EC00905, 1 mg/mL diluted 4000) and serum of a C57BL/6JOIaHsd (Harlan) immunized with three injections of the same ACI-DS-01 batch were used as positive control. After washing, plates were incubated with alkaline phosphatase (AP) conjugated anti-mouse IgG antibody (Jackson Immunoresearch West Grove, PA, USA, Cat. N°115-055-164 Lot 87821, vial diluted at 1/4000) for two hours at 37°C. After final washing, plates were incubated during 2 hours and a half with AP substrate (pNPP) and read at 405 nm using an ELISA plate reader. Results are expressed as optical density (O.D).

### 1.5 Behavioral testing after ACI-DS-01 immunization

All mice were exposed to the same series of behavioral tests starting at around 8 months old of age. Each mouse was handled for 10 min, twice a day, during the 7 days that preceded testing and for 3 days in between tests. The tests were performed at the indicated days (according to the last immunization) and in the following order: locomotor activity (starting day 11), novel object recognition task with 24 h delay (starting day 18), T-maze (starting day 25), and contextual fear conditioning test (starting day 34). All behavioral tests took place during the day-light cycle between 7:00 A.M. and 7:00 P.M. and were performed at room temperature (22°C). On the day of testing, mice were kept in their home cages during the day-light phase in the same experimental room for 2 h for habituation. As an indicator of anxiety during each test, the number of fecal pellets and urine drops was also measured. To minimize olfactory cues from the previous trial, each apparatus was thoroughly cleaned with 10% ethanol after each animal occupation. Handling and all behavioral tests were performed in a blinded manner, not revealing the genotype and treatment to the investigator handling the mice.

### 1.5.1 Spontaneous locomotor activity test after ACI-DS-01 immunization

Spontaneous locomotor activity was monitored using Plexiglas activity chambers (model MED-OFA-MS; Med Associates) (27.9 x 27.9 x 20 cm) and activity monitor software (Activity Monitor, version 4.3.6). As described in Belichenko et al., (Beliche*nko et al.,* 2009;Beliche*nko et al.,* 2007), mice were placed into the center of the chamber under bright ambient light conditions and activity was monitored for 10 min in three separate trials. Averages were determined for total distance, velocity, total activity time, total activity counts and vertical activity.

### 1.5.2 Novel object recognition task after ACI-DS-01 immunization

We used the Bevins and Besheer protocol (Bevins and Besheer, 2006;O'Doherty *et al.,* 2005): a one-trial non-matching-to-sample learning task to study recognition memory for two sample objects with one environment to study learning and memory with a 24 h delay. Before testing, mice were habituated in a black Plexiglas chamber (31 x 24 x 20 cm) during 10 min for 2 consecutive days under dimmed ambient light conditions. Activity of mice at the age of 8 months during the object recognition task was recorded with a video camera. First, two identical objects were placed in the chamber, as previously described (Bevins and Besheer, 2006;O'Doherty *et al.,* 2005). A mouse was placed at the mid-point of the wall opposite the sample objects. After 10 min exploring the objects, the mouse was returned to the colony for 24 h. To test for object recognition, one familiar object and one novel object were placed in the chamber and the mouse was again placed in the chamber for 3 min to explore the objects. Object recognition was measured in a single trial. Data were determined using direct observations of video recordings. Results were: 1) average total exploration time of the sample objects, both novel and familiar, and 2) discrimination ratio (novel object interaction/total interaction with both objects).

### 1.5.3 T-maze testing after ACI-DS-01 immunization

Mice at age 8 months were used. We used a modified Deacon and Rawlins protocol (Deacon and Rawlins, 2006) and a continuous alternation task in a T-maze to evaluate hippocampal function. The maze was made of opaque acrylic glass (Plexiglas) as described (Deacon and Rawlins, 2006) with an additional sliding door at the beginning of the start arm. During the test, a mouse was placed at the beginning of the start arm, with its back to the closed sliding door. After all doors were opened, the mouse ran down the start arm to choose either the right or left goal arm. After all four legs of the mouse had entered one goal arm, the sliding door to another goal arm was closed for 5 s, and then all sliding doors were opened again, allowing the mice to go back to the start arm. T-maze activity was monitored until the mice finished 10 alternations. This procedure was repeated for 3 consecutive days, for a total of 30 trials. The spontaneous alternation score was defined as the number of left-right and right-left alternations, expressed as a percentage of the total number of possible alternations during the session. Results were averaged for both alternation scores and time spent.

### 1.5.4 Contextual fear conditioning test after ACI-DS-01 immunization

Contextual and cued fear conditioning was conducted for evaluation of fear-dependent learning and retrieval. The test was performed using chambers from Coulbourn Instruments (Whitehall, PA, USA). On the first day animals were placed in a chamber (Context A) for 3 minutes for baseline recording, followed by five tone-shock pairings. The shock (0.5 mA, 2 sec) was delivered following the tone (70 dB, 2 kHz, 20 sec) in each conditional /unconditional stimulus pairing. On the second day, a novel chamber (Context B; new room, new olfactory environment, new texture of floor, blue plastic inserts for walls, extra source of blue light, and visual cues) was used for cued testing. Following a 3-minute pre-tone period, three tones without shocks were presented to animals during a 3-minute testing period. On the last day of the experiment, the mice were placed in Context A for 5 minutes without any conditional or unconditional stimulus (Saxe *et al.,* 2006). Freezing was defined as the complete lack of motion for a minimum of 0.75 seconds as measured by FreezeFrame software (Actimetrics, Evanston, IL). The percentage of freezing in each period was reported.

### 1.6 Measuring body and brain weights after ACI-DS-01 immunization

After all behavioral tests, mice were deeply anesthetized with sodium pentobarbital (200 mg/kg i.p.) (Abbott Laboratories), weighed, and transcardially perfused for 1 min with 0.9% sodium chloride (10 ml) and then for 10 min with 4% paraformaldehyde in 0.1 M PBS, pH 7.4 (100 ml). After perfusion, the brain was immediately removed. The weight of the brain (including the olfactory bulbs, cortex, hippocampus, cerebellum, brainstem and the cervical spinal cord through C1-C2) was recorded. The brain was then placed in fixative until further use.

### 1.7 Immunofluorescence for inflammatory reaction after ACI-DS-01 immunization

Brain sections were pre-incubated with 5% non-fat milk in 0.1M PBS with 0.3% Triton X-100. Sections were then incubated overnight at 4 °C with rabbit anti-cow glial fibrillary acidic protein (GFAP) antibody (DAKO, Glostrup, Denmark) at a dilution of 1:500, or with polyclonal rat anti-CD45 (Pharmingen) at a dilution of 1:5000. The following incubations (1 h each and at room temperature) were the done; with a biotinylated donkey anti-rabbit secondary antibody (1:200; Jackson ImmunoResearch Labs, West Grove, PA, USA) and with fluorescein isothiocyanate (FITC)-conjugated streptavidin (1:500; Jackson ImmunoResearch Labs). A rinse with PBS (3 times, each 20 min) was done between the incubations described above. Sections were mounted onto microscope glass slides and coverslipped using 90% glycerol in 0.1M phosphate buffer, pH 7.4. To control for specificity of antibody staining, selected sections were submitted to the same protocol but without including the primary antibodies. Immunofluorescence was not observed in control sections. Slices were examined and scanned in a Radiance 2000 (Bio-Rad, Hertfordshire, UK) confocal microscope attached to a Nikon Eclipse E800 fluorescence microscope. The laser was an argon/krypton mixed gas laser with excitation wavelengths for FITC at 488 nm (λ). LaserSharp software (Bio-Rad) was used to establish optimal conditions for collecting images. The optimal conditions for confocal imaging of GFAP- immunoreactivity (IR) or CD45-IR were the following: the lens was a 20x objective (Nikon; Plan Apo 20x/0.75); laser power was 10% or 20%; gain was 34.7; offset was 0.0; the zoom factor was 3; scan speed was 500 lines/s; each optical section was scanned three times and Kalman filtering was then employed to reduce noise; the size of the image was 512x512 pixels and the pixel size was 0.48x0.48 um.

### 1.8 Statistical analysis after ACI-DS-01 immunization

Data are shown as mean ± standard deviation (SD) or standard error of mean (SEM). Statistical analysis was done by unpaired t-test, two tailed. A probability of p<0.05 was considered significant.

### Example 2 Immunization with ACI-DS-01 produced anti-mouse Aβ antibodies

The liposomal vaccine ACI-DS-01 was prepared according to the SupraAntigen™ methodology (WO 2005/081872, WO2007/068411) with tetrapalmitoylated mouse Aβ 1-15 peptide (Palm1-15), H-Lys(Pal)-Lys(Pal)-Asp-Ala-Glu-Phe-Gly-His-Asp-Ser-Gly-Phe-Glu-Val-Arg-His-Gln-Lys(Pal)-Lys(Pal)-OH embedded into liposomes along with monophosphoryl lipid A (MPLA), (Figure 1). Six doses of ACI-DS-01 were administered subcutaneously and bi-weekly into Ts65Dn male mice and age-matched control mice (2N). The analysis of plasma collected after the 4^{th} dose, showed robust IgG titers against mouse Aβ40 or Aβ42 in the immunized mice of both Ts65Dn and 2N groups (Figure 2A and 2B). The vaccine-induced antibodies remained elevated even 40 days after the last immunization. No titers were detected in mice immunized with empty liposome without the antigen (Figure 2A and 2B). Thus, ACI-DS-01 was able to break Aβ self-tolerance in the DS mouse model.

Subclasses of IgG were analyzed in plasma of mice immunized with ACI-DS-01 following the 4^{th} immunization. The ELISA done with Aβ40 showed that the Ts65Dn group had higher IgG2a titers than the 2N group (Figure 2D) while there was no difference between the two groups for IgG1 and IgG2b titers (Figure 2C and 2E) (One-way ANOVA, Tukey posthoc; P = 0.05). The IgG3 titers were lower in the Ts65Dn group (Figure 2F) (One-way ANOVA, Tukey posthoc; P<0.001). IgM titers were slightly, but significant, lower in the Ts65Dn mice in comparison to 2N mice (Figure2G). There (One-way ANOVA, Tukey posthoc; P = 0.05). Similar results were obtained in ELISA performed with Aβ42 (data not shown).

The lower levels of a few of the titers mentioned above in Ts65Dn mice are unlikely to be due to the immune response capacity of the Ts65Dn since the anti-MPLA IgG level was comparable in all mice (Figure2H).

### Example 3 Immunization with ACI-DS-01 restored the memory deficit of Ts65Dn mice

Ts65Dn mice exhibit many features of DS cognitive abnormalities such as memory impairment and abnormal activities. To investigate the efficacy of the ACI-DS-01 vaccine, a battery of behavioral tests was conducted two weeks after the last immunization. Mice performed the tests in the following order; open field, object recognition, T-maze and fear conditioning.

The analysis of the open field test showed that Ts65Dn mice have a significant higher spontaneous locomotor activity in comparison to 2N mice (data not shown). Following immunization with ACI-DS-01, Ts65Dn mice continued to be significantly more active than ACI-DS-01 treated 2N mice (Fig.3A, t test unpaired one-tailed; p = 0.0004). In the T-maze, 2N and Ts65Dn mice showed no significant difference in the alteration ratio or in the alteration duration, neither before treatment nor post treatment (data not shown).

The spatial memory capacity was measured in the object recognition (ORT). The Ts65Dn mice exert a weak discrimination ratio and thus recognize poorly the novel object. ACI-DS-01 treated Ts65Dn mice showed a higher discrimination ratio indicating that treatment led to a significant improvement of memory (Fig. 3B, t test unpaired one-tailed; p = 0.03. one way ANOVA, genotype, P = 0.12, vaccine, P =0.002. Interaction P = 0.54). Interestingly, the same results were observed in the 2N group.

The fear conditioning test consists of training, cued and contextual sessions. The same level of freezing was observed in all groups during the training or the cued session (Figure 3C). During the contextual session, Ts65Dn mice treated with empty vaccine (only the liposome, no antigen) showed a lower percent of freezing than the control 2N mice. In contrast, after immunization Ts65Dn mice demonstrated an enhanced freezing and reached a comparable level to the 2N mice (t-test unpaired one-tailed; P = 0.04. (one way ANOVA, genotype, P = 0.01, vaccine, P =0.16. Interaction P = 0.33). This suggests that the immunization was efficient and enhanced the memory capacity of Ts65Dn mice.

In summary, these results indicate that ACI-DS-01 treatment can restore the memory deficit of Ts65Dn mice.

### Example 4 Mechanism of ACI-DS-01 vaccine for improving the cognitive deficiency

To address the question about the effect of the ACI-DS-01 induced antibodies on the level of Aβ, ELISA was performed using protein extracts of the hippocampus, the cortex and the cerebellum. None of these regions showed a significant difference on Aβ levels following the treatment with ACI-DS-01; neither for Aβ40 nor for Aβ42 (Figure 4A). It is worthwhile to note that the ratio Aβ-40/42 in the cortex and the cerebellum correlated significantly with a higher recognition index (RI) (Figure 4B and 4C. Cortex; Pearson r correlation = -0.3248, P = 0. 0.03. cerebellum; Pearson r correlation = -0.4127, P = 0.009). These results suggest that Aβ-40/42 might be responsible of the cognitive deficit. Further analysis showed that Aβ42 in the plasma was undetectable. In contrast, an increased level of Aβ40 in the plasma seemed to be associated with the higher values of anti-Aβ IgG measured in the group of ACI-DS-01 treated Ts65Dn mice (Figure 4D; Pearson r correlation = 0.51, P =0.09). Moreover, the RI correlated with anti-Aβ IgG titers (Figure 4E; Pearson r correlation = 0.3154, P =0.007).

These results suggest that ACI-DS-01-induced antibodies may clear Aβ from the brain to the plasma which then may lead to memory amelioration.

### Example 5 Morphology of neurons in Mouse Models of Down Syndrome

The size, the number and optical density of cholinergic neuron in the basal forebrain (BFCN) were analyzed. Two sections from BFCN level were stained with ChAT antibody (Millipore, Cat # AB144P, lot # 2010060). Medial septum (MS) area was scanned on confocal microscope for quantitative evaluation. The following parameters were calculated 1) density of ChAT+ cells in MS; 2) area of ChAT+ individual neurons, and 3) average of optical density of ChAT in individual neurons. Cholinergic neurons in medial septum were imaged and ImageJ software was used to count number of neurons, reveal optical density of staining and outlining of cell body area.
Results: Number of ChAT+ cells in medial septum and optical density was similar in 2N and Ts65Dn mice treated with empty vaccine (Figure 9A and 9B).

Number of ChAT+ cells in medial septum was similar in ACI-DS-01 treated 2N and Ts65Dn mice (Figure 9A) (2N-Empty = 27.5 ± 7.2 per 100 microm section; Ts65Dn- Empty = 23.9 ± 7.0; 2N-ACI-DS-01 = 36.6 ± 4.6; Ts65Dn-ACI-DS-01 = 20.9 ± 6.6). Optical density of staining in individual ChAT+ cells in medial septum was also similar in ACI-DS-01 treated 2N and Ts65Dn mice (Figure 9B) (2N- Empty = 79.9 ± 3.5 arbitrary unit; Ts65Dn- Empty = 74.7 ± 2.6; 2N-ACI-DS-01 = 71.9 ± 2.6; Ts65Dn-ACI-DS-01 = 75.8 ± 4.5). The area of ChAT+ cell body in medial septum was lower in Ts65Dn than 2N mice (Figure 9C).The area of ChAT+ cell body in medial septum of Ts65Dn- ACI-DS-01 treated mice was significantly increased in comparison to empty treated Ts65Dn-veh (Ts65Dn- Empty = 179 ± 6 microm²; Ts65Dn-ACI-DS-01 = 201 ± 8, p = 0.031). 2N and Ts65Dn ACI-DS-01 treated mice had similar area of cell body (2N-ACI-DS-01 = 208 ± 8 microm²; Ts65Dn-ACI-DS-01 = 201 ± 8, p = 0.55) (Figure 9C). This point out on recovering of cell body area in ChAT+ cells in medial septum upon ACI-DS-01 treatment. Equivalently, these results are an indication of the safety of the ACI-DS-01 vaccine since the number of ChAT+ cells did not change after immunization with the vaccine. These morphological studies showed less atrophy of neurons after ACI-DS-01 immunization indicating that this vaccine could be used for prevention of neurodegeneration.

### Example 6 The safety of the liposomal vaccine ACI-DS-01

To determine if immunization with ACI-DS-01 may induce safety concerns, body weight, general observation, and inflammation markers in mice were recorded. Before treatment, Ts65Dn mice had a significantly lower body weight than 2N mice (ANOVA, Tukey posthoc, p = 0.001). Immunization did not alter significantly the body weight (Figure 5A). Brain weights were similar in all four groups of mice (Figure 5A). The immunohistochemistry results showed no difference in the number of glial fibrillary acidic protein (GFAP)-positive astroglial cells or of the CD45-positive microglial cells. The obtained results were similar in the cortex and hippocampus (Figure 5). These results indicate that ACI-DS-01 vaccination did not induce an inflammatory response.

To verify the specificity of ACI-DS-01 induced antibodies, a cross reaction study was conducted. The brain sections of 2N and Ts65Dn were stained with the antiserum of ACI-DS-01-treated 2N mice.

In summary, these findings indicate that ACI-DS-01 is a safe vaccine.

### Example 7: General Methodology for experiments with ACI-DS-02 and ACI-DS-03 vaccines

### 7.1 Immunization with ACI-DS-02 and ACI-DS-03 vaccine

Ts65Dn mice, males (Jax laboratory B6EiC3Sn.BLiA-Ts(1716)65Dn/DnJ) 4±0.3 months old at the start of the study, received subcutaneous (s.c.) immunizations with 200µl of ACI-DS-02 (18.6 µg per dose of dipalmitoylated Aβ peptide 22-39) (n= 6 Ts65Dn mice) or PBS (n= 4 Ts65Dn mice). A total of four injections were done at day 1, 14, 28 and 42. Tail bleedings were performed one week following each immunization; day 7, 21, 35 and 49.

The same procedure was done for the immunization with the vaccine ACI-DS-03 (38.6 µg per dose of Pal 14-29, tetrapalmitoylated Aβ peptide 14-29) (n= 7 Ts65Dn mice) or PBS (n= 4 Ts65Dn mice).

Control littermate mice (n = 7 2N-mice) received similar injections but with PBS.

### 7.2Quantification of mouse Aβ-specific antibodies

Aβ1-42-specific IgG responses were determined by ELISA. Briefly, plates were coated with 10 µg/mL of human Aβ1-42 (Bachem H1368, lot: 1000255) overnight at 4°C. After washing with PBS-0.05% Tween 20 and blocking with 1% BSA, serial dilutions of plasma were added to the plates and incubated at 37°C for two hours. The antibody anti-Aβ 6E10 (6E10 from Covance SIG-39320, lot: 09GC01254 vial a, 1 mg/ml diluted 1000x) was used as positive control. After washing, plates were incubated with alkaline phosphatase (AP) conjugated anti-mouse IgG antibody (Jackson Immunoresearch West Grove, PA, USA, Cat. N°115-055-164 Lot 87821, vial diluted at 1/4000) for two hours at 37°C. After final washing, plates were incubated during 2 hours and a half with AP substrate (pNPP) and read at 405 nm using an ELISA plate reader. Results are expressed as optical density (O.D).

### 7.3 Behavioral testing

All mice were exposed to the same series of behavioral tests starting at around 6±0.3 months old of age. All behavioral tests took place during the day-light cycle between 7:00 A.M. and 7:00 P.M. and were performed at room temperature (22°C).

### 7.3.1 Spatial object recognition task

Before testing, mice were habituated in a grey polypropylene chamber (Diameter 40 cm, height 32.4 cm) during 10 min without any object under dimmed ambient light conditions. In the following day, and during the learning session, two identical objects (cube of Lego or glass black bottle) were presented (10 min) on one side of the maze. The retention test was done 3 hours post learning, one of the identical objects is moved to the opposite side of the maze and is presented during 10 min. All sessions were recorded with the video tracking software Ethovision XT 8.0. The measured parameters included the average total exploration time of the sample objects, both novel and familiar, and discrimination ratio (novel object interaction/total interaction with both objects).

### 7.3.2 Water Maze memory task

The water maze was conducted to analyze the long term memory. As a habituation test, mice were subjected in the first day to a water maze (diameter: 165 cm, height: 64 cm) with a visible, cued platform (diameter 11 cm, height: 37 cm). This test consisted in 5 trials of each of 120 seconds with 30 minutes inter-trial interval. The final 6th trial was conducted without the visible, cued platform. The following day, mice were subjected to 4 days training with a hidden platform. On each day, mice performed 6 trials each of 120 seconds and with inter-trial interval 30 minutes. At the beginning of a trial, a mouse is placed in the water facing the edge of the tank, at one of the 4 starting points (North-East, South-East, South-West, and North-West). The starting points are changed from trial to trial, while the escape platform is fixed (West quadrant) throughout the experiment. If the mouse finds the platform within 120 sec, it is allowed to remain on the platform for 15 sec. If it fails to find the platform within 120 sec, the trial is stopped and the mouse is gently guided to the platform and left there for 15 sec. The measured parameters included the escape latency time (s) spent in the different parts of the maze, number of entries into each quadrant of the maze, distance (cm) travelled into each quadrant of the maze, distance (cm) to target area, % time in each quadrant, % time in the extended target platform annulus during probe trial, number of crossing of the extended target platform annulus during probe trial and velocity (cm/s).

### 7.3.3 Contextual fear conditioning test

Contextual and cued fear conditioning was conducted for evaluation of fear-dependent learning and retrieval. The test was performed using chambers from Med Associates Instruments. On the first day mice were placed in a chamber (Context A) for 2 minutes for baseline recording, followed by four tone-shock pairings (conditioned-stimuli, CS). The shock 30 seconds tone stimulus (5000 Hz, 80 dB) co-terminated with 2 seconds foot-shock (0.7 mA) in each conditional /unconditional stimulus pairing. On the second day, the mice were placed in context A for 5 minutes without any conditional or unconditional stimulus. On the last day of the experiment, a novel chamber (Context B; new room, new olfactory environment, new texture of floor, blue plastic inserts for walls, extra source of blue light, and visual cues) was used for cued testing. Following a 2-minute pre-tone period, three tones without shocks were presented to animals during 8-minute testing period. Freezing was defined as the complete lack of motion for a minimum of 0.75 seconds. The percentage of freezing in each period was reported.

### 7.4 Immuno-staining of amyloid plagues in human DS brain samples with ACI-DS-02 and ACI-DS-03 vaccines induced anti-Aβ antibodies

Brain sections from DS people were stained with vaccine derived anti-Aβ antibodies in sera obtained from immunized mice with the ACI-DS-02 vaccine. A pool of sera from five c57BL/6 mice immunized with ACI-DS-02 vaccine was used at the dilution of 1/100 for the procedure of immuno-staining. Briefly, cryosections of 10µm of a DS individual of 59 years old was incubated in 4% paraformaldehyde (Sigma-Aldrich, 252549) for 20 minutes. Following the incubation in 70% formic acid (Merck, 1.00264.1000) and 10% Triton X-100 (Sigma, 234729) each for 15 minutes, sections were blocked for 2 hours in 10% Normal goat Serum (NGS, Invitrogen, 6210072) in PBST (PBS plus 0.5% Triton X-100). The incubation with the primary antibody (sera of immunized mice) in PBST with 10% NGS was done overnight. The next day, and after rinsing in PBSA, sections were incubated with Alexa Fluor 488 conjugated AffiniPure Goat Anti-Mouse IgG (Jackson ImmunoResearch, 115-545-146). Sectiones were mounted in ProLong Gold (Invitrogen, P36931). As positive control, the primary antibody anti-Aβ 6E10 was used (Covance, SIG-39320, anti- Aβ against N-terminal end of Aβ using a dilution of 1:10000). Sera of c57BL/6 mice immunized with PBS were used as negative control. Sera of c57BL/6 mice immunized with ACI-24 were used as a control. ACI-24 vaccine corresponds to ACI-DS-01 vaccine with the difference that the antigen used in ACI-24 is a human Aβ 1-15 sequence, while ACI-DS-01 vaccine contains mouse Aβ 1-15 sequence as the antigen (for three amino acid difference in those two antigens see Figure 1).

### 7.5 Statistical analysis

Data are shown as mean ± standard deviation (SD) or standard error of mean (SEM). Statistical analysis was done by unpaired t-test, two tailed or ANOVA using prism pad graph version 5. A probability of p<0.05 was considered significant.

### Example 8 Immunization with ACI-DS-02 produced anti-human Aβ antibodies

Four doses of ACI-DS-02 were administered subcutaneously and bi-weekly into Ts65Dn male mice and age-matched control mice 2N. The analysis of plasma collected 7 days after each dose, showed IgG titers against human Aβ42 in the immunized mice of both Ts65Dn and 2N groups (data not shown). The IgG titers of Ts65Dn mice immunized with ACI-DS-02 were significantly different from those immunized with PBS at day 21 and 35 (one way ANOVA, Tukey posthoc, P = 0.01 and 0.05 respectively). Thus, ACI-DS-02 was able to break Aβ self-tolerance in the DS mouse model.

The IgM titers were analyzed in plasma of mice immunized with the ACI-DS-02 vaccine. The ELISA done with Aβ42 showed that the immunized Ts65Dn group had higher IgM titers than the group immunized with PBS at day 7, day 35 and day 49 (one way ANOVA, Tukey posthoc, P<0.01, P < 0.05 and P<0.01 respectively, data not shown.)

### Example 8 ACI-DS-02 derived anti-human Aβ antibodies recognize DS amyloid plaques

The immuno-staining experiment showed the ACI-DS-02 derived antibodies bind to amyloid plaques in the brain samples of DS people (Figure 6). The staining was similar to the obtained staining with 6E10, a commercial available anti-amyloid beta antibody, and with ACI-24 derived antibody. ACI-24 vaccine corresponds to ACI-DS-01 vaccine with the difference that the antigen used in ACI-24 is a human Aβ 1-15 sequence, while ACI-DS-01 vaccine contains mouse Aβ 1-15 sequence as the antigen (for three amino acid difference in those two antigens see Figure 1). No staining was observed when a section was incubated with sera of mice immunized with PBS. This result indicates that immunization with the ACI-DS-02 vaccine induces antibodies that recognize amyloid plaques in the brain of DS people.

### Example 9 ACI-DS-02 enhanced associative learning in the fear conditioning test

During the conditioning test, Ts65Dn mice immunized with ACI-DS-02 vaccine showed a trend to higher levels of freezing after receiving each conditioned stimulus (Figure 7A). The enhanced freezing was significantly different from the PBS immunized Ts65Dn group only at the third conditioned stimulus (CS3) (two way ANOVA, time, P<0.0001, a trend for vaccine (P = 0.09), Bonferroni posttests showed P = 0.05 at the CS3).

The learning session is known to be mediated by synaptic plasticity-dependent mechanism (Johansen 2011). However, previously acquired fear memory (context, cued and extinction sessions) is known to be mediated by a different neuronal mechanism. This result suggests that the ACI-DS-02 immunization enhanced acquisition/training phase of fear conditioning during which learning occurs.

### Example 10 Immunization with ACI-DS-03 produced anti-human Aβ antibodies

Four doses of ACI-DS-03 were administered subcutaneously and bi-weekly into Ts65Dn male mice and age-matched control mice 2N. The analysis of plasma collected 7 days after each dose, showed IgG titers against human Aβ42 in the immunized mice of both Ts65Dn and 2N groups (data not shown). The IgG titers of Ts65Dn mice immunized with ACI-DS-03 were significantly different from those immunized with PBS at day 21 (one way ANOVA Tukey posthoc, P = 0.05). Thus, ACI-DS-03 was able to break Aβ self-tolerance in the DS mouse model.

The IgM titers were analyzed in plasma of mice immunized with ACI-DS-03 vaccine. The ELISA done with Aβ42 showed that the immunized Ts65Dn group had higher IgM titers than the group immunized with PBS at day 7 and 49 (one way ANOVA, Tukey posthoc, P<0.001 and P<0.01 respectively (data not shown).

### Example 11 ACI-DS-03 derived anti-human Aβ antibodies recognize DS amyloid plaques

The immuno-staining experiment showed the ACI-DS-03 derived antibodies bind to amyloid plaques in the brain of DS people (Figure 8). The staining was similar to the obtained staining with 6E10, a commercial available anti-amyloid beta antibody, and with ACI-24 derived antibody. ACI-24 vaccine corresponds to ACI-DS-01 vaccine with the difference that the antigen used in ACI-24 is a human Aβ 1-15 sequence, while ACI-DS-01 vaccine contains mouse Aβ 1-15 sequence as the antigen (for three amino acid difference in those two antigens see Figure 1). No staining was observed when section was incubated with sera of mice immunized with PBS. This result indicates that immunization by ACI-DS-03 vaccine induces antibodies that recognize amyloid plaques in the brain of DS people.

### Example 12 Attenuated Total Reflectance InfraRed spectroscopy

Liposomal samples were analyzed as a D₂O hydrated thin-film from 15 µl dried on the ZnSe crystal. Spectra were obtained with a BRUKER TENSOR 27 FTIR spectrometer equipped with a liquid nitrogen cooled mercury-cadmium-telluride detector and coupled to a BioATR-II device. For each spectrum 2000 scans were collected using the clean ATR crystal as background with a resolution of 2 cm-1, averaged and FT processed. The upper and the lower frequency folding limits were 4000 cm-1 and 900 cm-1. The sample chamber was continuously purged with dried air and all measurements were performed at 25 °C. Then acquired spectra were cut in the amide I region, baseline corrected and labeled. Narrowing techniques [second derivative and fourier self deconvolution (FSD)] were applied in order to reveal the overlapping components of the broad amide I band. A qualitative assignment_of the major secondary conformation was done from the main detected bands using the following frequency ranges in D₂O: b-sheet 1613-1637 cm⁻¹, random coil 1637-1646 cm⁻¹, a-helix/loops 1646-1662 cm⁻¹, b-turns 1662-1682 cm⁻¹, anti-parallel b-sheet 1682-1698 cm⁻¹. A quantitative analysis was done by curve-fitting with the corresponding application in OPUS software, using the bands identified in the 2^{nd} derivative and FSD spectra as initial values for the individual components. Component peaks were assumed to be of mixed Gaussian-Loretzian shape. The iteration process was carried out by fixing the frequencies and allowing the peak widths, intensities and shapes to vary and then for the second iteration process, the frequencies were also allowed to vary. Secondary structure estimates were made by calculating the % area of the total amide I region for each secondary structure band, assuming equal molar absorptivities of different components.

### Reference List

1. Antonarakis,S.E., Lyle,R., Dermitzakis,E.T., Reymond,A., Deutsch,S., 2004. Chromosome 21 and down syndrome: from genomics to pathophysiology. Nat. Rev. Genet. 5, 725-738.
2. Ballard,C., Gauthier,S., Corbett,A., Brayne,C., Aarsland,D., Jones,E., 2011. Alzheimer's disease. Lancet. 377, 1019-1031.
3. Belichenko,N.P., Belichenko,P.V., Kleschevnikov,A.M., Salehi,A., Reeves,R.H., Mobley,W.C., 2009. The "Down syndrome critical region" is sufficient in the mouse model to confer behavioral, neurophysiological, and synaptic phenotypes characteristic of Down syndrome. J Neurosci. 29, 5938-5948.
4. Belichenko,P.V., Kleschevnikov,A.M., Salehi,A., Epstein,C.J., Mobley,W.C., 2007. Synaptic and cognitive abnormalities in mouse models of Down syndrome: exploring genotype-phenotype relationships. J Comp. Neurol. 504, 329-345.
5. Bevins,R.A., Besheer,J., 2006. Object recognition in rats and mice: a one-trial non-matching-to-sample learning task to study 'recognition memory'. Nat. Protoc. 1, 1306-1311.
6. Davisson,M.T., Schmidt,C., Reeves,R.H., Irving,N.G., Akeson,E.C., Harris,B.S., Bronson,R.T., 1993. Segmental trisomy as a mouse model for Down syndrome. Prog. Clin. Biol. Res. 384, 117-133.
7. Deacon,R.M., Rawlins,J.N., 2006. T-maze alternation in the rodent. Nat. Protoc. 1, 7-12.
8. Gilman,S., Koller,M., Black,R.S., Jenkins,L., Griffith,S.G., Fox,N.C., Eisner,L., Kirby,L., Boada,R.M., Forette,F., Orgogozo,J.M., 2005. Clinical effects of A{beta} immunization (AN1792) in patients with AD in an interrupted trial. Neurology.
9. Hunter,C.L., Bimonte-Nelson,H.A., Nelson,M., Eckman,C.B., Granholm,A.C., 2004. Behavioral and neurobiological markers of Alzheimer's disease in Ts65Dn mice: effects of estrogen. Neurobiol. Aging. 25, 873-884.
10. Johansen I.P., Cain C.K.,Ostroff L.E and LeDoux J.E., 2011. Molecular Mechanisms of Fear Learning and Memory. Cell 147
11. Lee,M., Bard,F., Johnson-Wood,K., Lee,C., Hu,K., Griffith,S.G., Black,R.S., Schenk,D., Seubert,P., 2005. Abeta42 immunization in Alzheimer's disease generates Abeta N-terminal antibodies. Ann. Neurol. 58, 430-435.
12. Lemere,C.A., Blusztajn,J.K., Yamaguchi,H., Wisniewski,T., Saido,T.C., Selkoe,D.J., 1996. Sequence of deposition of heterogeneous amyloid beta-peptides and APO E in Down syndrome: implications for initial events in amyloid plaque formation. Neurobiol Dis. 3, 16-32.
13. Liu,F., Grundke-Iqbal,I., Iqbal,K., Oda,Y., Tomizawa,K., Gong,C.X., 2005. Truncation and activation of calcineurin a by calpain I in Alzheimer disease brain. J. Biol. Chem. 280, 37755-37762.
14. Liu,F., Liang,Z., Wegiel,J., Hwang,Y.W., Iqbal,K., Grundke-Iqbal,I., Ramakrishna,N., Gong,C.X., 2008. Overexpression of Dyrk1A contributes to neurofibrillary degeneration in Down syndrome. FASEB J. 22, 3224-3233.
15. Moncaster,J.A., Pineda,R., Moir,R.D., Lu,S., Burton,M.A., Ghosh,J.G., Ericsson,M., Soscia,S.J., Mocofanescu,A., Folkerth,R.D., Robb,R.M., Kuszak,J.R., Clark,J.I., Tanzi,R.E., Hunter,D.G., Goldstein,L.E., 2010. Alzheimer's disease amyloid-beta links lens and brain pathology in Down syndrome. PLoS One 5, e10659.
16. Monsonego,A., Maron,R., Zota,V., Selkoe,D.J., Weiner,H.L., 2001. Immune hyporesponsiveness to amyloid beta-peptide in amyloid precursor protein transgenic mice: implications for the pathogenesis and treatment of Alzheimer's disease. Proc. Natl. Acad. Sci. U. S. A 98, 10273-10278.
17. Netzer,W.J., Powell,C., Nong,Y., Blundell,J., Wong,L., Duff,K., Flajolet,M., Greengard,P., 2010. Lowering beta-amyloid levels rescues learning and memory in a Down syndrome mouse model. PLoS One 5, e10943.
18. Nicolau,C., Greferath,R., Balaban,T.S., Lazarte,J.E., Hopkins,R.J., 2002. A liposome-based therapeutic vaccine against beta -amyloid plaques on the pancreas of transgenic NORBA mice. Proc. Natl. Acad. Sci. U. S. A 99, 2332-2337.
19. O'Doherty,A., Ruf,S., Mulligan,C., Hildreth,V., Errington,M.L., Cooke,S., Sesay,A., Modino,S., Vanes,L., Hernandez,D., Linehan,J.M., Sharpe,P.T., Brandner,S., Bliss,T.V., Henderson,D.J., Nizetic,D., Tybulewicz,V.L., Fisher,E.M., 2005. An aneuploid mouse strain carrying human chromosome 21 with Down syndrome phenotypes. Science. 309, 2033-2037.
20. Prasher,V.P., 1993. Down's syndrome, longevity, and Alzheimer's disease. Br. J Psychiatry. 162, 711.
21. Prasher,V.P., 2004. Review of donepezil, rivastigmine, galantamine and memantine for the treatment of dementia in Alzheimer's disease in adults with Down syndrome: implications for the intellectual disability population. Int. J Geriatr. Psychiatry. 19, 509-515.
22. Rafii,M.S., 2010. The pulse of drug development for Alzheimer's disease. Rev. Recent. Clin. Trials. 5, 57-62.
23. Saxe,M.D., Battaglia,F., Wang,J.W., Malleret,G., David,D.J., Monckton,J.E., Garcia,A.D., Sofroniew,M.V., Kandel,E.R., Santarelli,L., Hen,R., Drew,M.R., 2006. Ablation of hippocampal neurogenesis impairs contextual fear conditioning and synaptic plasticity in the dentate gyrus. Proc. Natl. Acad. Sci. U. S. A. 103, 17501-17506.
24. Stanton L.R, Coetzee R.H . Down's syndrome and dementia. 10, 50-58. 2004. Advances in Psychiatric Treatment. Ref Type: Generic
25. Stoltzner,S.E., Grenfell, T.J., Mori,C., Wisniewski,K.E., Wisniewski,T.M., Selkoe,D.J., Lemere,C.A., 2000. Temporal accrual of complement proteins in amyloid plaques in Down's syndrome with Alzheimer's disease. Am. J. Pathol. 156, 489-499.
26. Tapiola,T., Soininen,H., Pirttila,T., 2001. CSF tau and Abeta42 levels in patients with Down's syndrome. Neurology. 56, 979-980.
27. Weiner,H.L., Frenkel,D., 2006. Immunology and immunotherapy of Alzheimer's disease. Nat. Rev. Immunol. 6, 404-416.
28. Fernandez F, Morishita W, Zuniga E, Nguyen J, Blank M, Malenka RC and Garner CC (2007)
29. Hanney M, Prasher V, Williams N, Jones EL, Aarsland D, Corbett A, Lawrence D, Yu LM, Tyrer S, Francis PT, Johnson T, Bullock R and Ballard C (2012) Memantine for Dementia in Adults Older Than 40 Years With Down's Syndrome (MEADOWS): a Randomised, Double-Blind, Placebo-Controlled Trial. Lancet 379:528-536.
30. Salehi A, Delcroix J-D, Belichenko P.V., Zhan K; Wu C., Valletta J C, Takimoto-Kimura R, Kleschevnikov A M, Sambamurti K, Chung PP, Xia W, VillarA, Campbell AW, Kulnane LS, Nixon RA, Lamb BT, Epstein CJ, Stokin GB, Goldstein L.S.B, Mobley W.C. (2006) Increased App Expression in a Mouse Model of Down's Syndrome Disrupts NGF Transport and Causes Cholinergic Neuron Degeneration, Neuron, 51, 29-42.
31. Salehi A, Faizi M, Colas D, Valletta J, Laguna J, Takimoto-Kimura R., Kleschevnikov A., Wagner S. L., Aisen P., Shamloo M., MobleyW. C (2009) Restoration of Norepinephrine-Modulated Contextual Memory in a Mouse Model of Down Syndrome, Sci Transl Med 1, 7ra17.

## Claims

1. An antigenic peptide derived from amyloid beta protein for use in the treatment and/or alleviation and/or prevention of memory and/or cognitive impairments or abnormalities in subjects with Down's syndrome, wherein the antigenic peptide consists of residues Aβ1-15 and is modified by a palmitic acid bound to the N- and/or C-terminus of the peptide and reconstituted in a liposome and said subjects with Down's syndrome have not yet developed amyloid protein-associated plaques in the brain.

2. The antigenic peptide of claim 1, wherein the Aβ1-15 peptide is modified by four palmitic acids bound to the N- and/or C-terminus of the peptide.

3. The antigenic peptide of claim 1, wherein the Aβ1-15 peptide is modified by two palmitic acids bound to the N- and/or C-terminus of the peptide.

4. The antigenic peptide for use according to any one of claims 1-3 wherein said impairment or abnormality concerns an impairment of recognition memory, an impairment of contextual associative memory, an impairment in associative learning, an impairment of declarative memory for facts and events, episodic memory impairments, a language dysfunction, a visuospatial impairment, a decrease in executive functions, personality changes, emotional changes, apraxia, impairments of performing learned motor tasks, pyramidal and extrapyramidal findings as well as myoclonus or seizures, or a combination thereof.

5. The antigenic peptide for use according to any one of claims 1 to 4, wherein said subject with Down's syndrome is
(a) a young to middle-aged subject; or
(b) a middle-aged subject; or
(c) a young-aged subject; or
(d) a children-aged subject, or,
(e) a subject below age 65.

6. The antigenic peptide for use according to any one of claims 1 to 5, wherein said use
a) leads to an amelioration and/or restoration of memory; and/or
b) leads to an increase in the retention or a complete restoration of cognitive capacity in the treated subject; and/or
c) leads to enhancing or restoring memory and/or cognitive capacity; and/or
d) does not induce unwanted side effects.

7. The antigenic peptide for use according to any one of claims 1 to 6 for prevention of the development of Aβ-associated plaques in the brain.

8. A composition comprising the antigenic peptide as defined in any one of claims 1 to 7 in a therapeutically effective amount together with a pharmaceutically acceptable carrier and/or excipient for use in the treatment and/or alleviation and/or prevention of memory and/or cognitive impairments or abnormalities in subjects with Down's syndrome, wherein said subjects with Down's syndrome have not yet developed amyloid protein-associated plaques in the brain.

9. The composition for use according to claim 8, comprising an adjuvant.

10. The composition for use according to claim 9, wherein the adjuvant is lipid A.

11. The composition for use according to claim 10, wherein the adjuvant is a detoxified lipid A.

12. The composition for use according to claim 9, wherein the adjuvant is monophosphoryl or diphosphoryl lipid A, alum, calcium phosphate, interleukin 1, and/or microcapsules of polysaccharides and proteins.

13. The antigenic peptide for use according to any one of claims 1 to 7 or the composition for use according to any one of claims 8 to 12, for prevention of neurodegeneration in a subject with Down's syndrome.

14. The antigenic peptide for use according to any one of claims 1 to 7 or the composition for use according to any one of claims 8 to 12, for inducing an immune response against amyloid beta protein in a subject with Down's syndrome.

## Patentansprüche

1. Antigenes Peptid, das von Amyloid-Beta-Protein abgeleitet ist, zur Verwendung bei der Behandlung und/oder Linderung und/oder Vorbeugung von Gedächtnis-Beeinträchtigungen oder -Anomalien und/oder kognitiven Beeinträchtigungen oder Anomalien bei Individuen mit Down-Syndrom, wobei das antigene Peptid aus den Resten Aβ1-15 besteht und durch eine Palmitinsäure modifiziert ist, die an den N- und/oder C-Terminus des Peptids gebunden und in einem Liposom rekonstituiert ist, und wobei die Individuen mit Down-Syndrom noch keine mit Amyloid-Protein in Zusammenhang stehende Plaques im Gehirn entwickelt haben.

2. Antigenes Peptid nach Anspruch 1, wobei das Aβ1-15-Peptid durch vier Palmitinsäuren modifiziert ist, die an den N- und/oder C-Terminus des Peptids gebunden sind.

3. Antigenes Peptid nach Anspruch 1, wobei das Aβ1-15-Peptid durch zwei Palmitinsäuren modifiziert ist, die an den N- und/oder C-Terminus des Peptids gebunden sind.

4. Antigenes Peptid zur Verwendung nach einem der Ansprüche 1 bis 3, wobei die Beeinträchtigung oder Anomalie eine Beeinträchtigung des Erkennungsgedächtnisses, eine Beeinträchtigung des kontextuellen assoziativen Gedächtnisses, eine Beeinträchtigung des assoziativen Lernens, eine Beeinträchtigung des deklarativen Gedächtnisses für Fakten und Ereignisse, Beeinträchtigungen des episodischen Gedächtnisses, eine Sprachdysfunktion, eine visuellräumliche Beeinträchtigung, eine Abnahme der exekutiven Funktionen, Persönlichkeitsveränderungen, emotionale Veränderungen, Apraxie, Beeinträchtigungen des Ausführens von erlernten motorischen Aufgaben, pyramidale und extrapyramidale Befunde sowie Myoklonus oder Anfälle, oder eine Kombination davon betrifft.

5. Antigenes Peptid zur Verwendung nach einem der Ansprüche 1 bis 4, wobei das Individuum mit Down-Syndrom
(a) ein Individuum im jungen bis mittleren Alter; oder
(b) ein Individuum im mittleren Alter; oder
(c) ein Individuum im jungen Alter; oder
(d) ein Individuum im Kindesalter; oder
(e) ein Individuum jünger als 65 Jahre
ist.

6. Antigenes Peptid zur Verwendung nach einem der Ansprüche 1 bis 5, wobei die Verwendung
a) zu einer Verbesserung und/oder Wiederherstellung des Gedächtnisses führt; und/oder
b) zu einem Anstieg der Retention oder einer vollständigen Wiederherstellung der kognitiven Kapazität bei dem behandelten Individuum führt; und/oder
c) zu einer Verbesserung oder Wiederherstellung der Gedächtnis-Kapazität und/oder kognitiven Kapazität führt; und/oder
d) keine unerwünschten Nebenwirkungen induziert.

7. Antigenes Peptid zur Verwendung nach einem der Ansprüche 1 bis 6 zur Vorbeugung der Entwicklung von mit Aβ in Zusammenhang stehenden Plaques im Gehirn.

8. Zusammensetzung, umfassend das antigene Peptid wie in einem der Ansprüche 1 bis 7 definiert in einer therapeutisch wirksamen Menge zusammen mit einem pharmazeutisch verträglichen Träger und/oder Exzipienten, zur Verwendung bei der Behandlung und/oder Linderung und/oder Vorbeugung von Gedächtnis-Beeinträchtigungen oder - Anomalien und/oder kognitiven Beeinträchtigungen oder Anomalien bei Individuen mit Down-Syndrom, wobei die Individuen mit Down-Syndrom noch keine mit Amyloid-Protein in Zusammenhang stehende Plaques im Gehirn entwickelt haben.

9. Zusammensetzung zur Verwendung nach Anspruch 8, umfassend ein Adjuvans.

10. Zusammensetzung zur Verwendung nach Anspruch 9, wobei das Adjuvans Lipid A ist.

11. Zusammensetzung zur Verwendung nach Anspruch 10, wobei das Adjuvans ein detoxifiziertes Lipid A ist.

12. Zusammensetzung zur Verwendung nach Anspruch 9, wobei das Adjuvans Monophosphoryl- oder Diphosphoryl-Lipid-A, Alaun, Calciumphosphat, Interleukin 1 und/oder Mikrokapseln von Polysacchariden und Proteinen ist.

13. Antigenes Peptid zur Verwendung nach einem der Ansprüche 1 bis 7 oder Zusammensetzung zur Verwendung nach einem der Ansprüche 8 bis 12, zur Vorbeugung von Neurodegeneration in einem Individuum mit Down-Syndrom.

14. Antigenes Peptid zur Verwendung nach einem der Ansprüche 1 bis 7 oder Zusammensetzung zur Verwendung nach einem der Ansprüche 8 bis 12, zum Induzieren einer Immunantwort gegen Amyloid-Beta-Protein in einem Individuum mit Down-Syndrom.

## Revendications

1. Peptide antigénique dérivé d'une protéine bêta amyloïde destiné à être utilisé dans le traitement et/ou le soulagement et/ou la prévention de détériorations ou d'anomalies de la mémoire et/ou cognitives chez des sujets atteints du syndrome de Down, le peptide antigénique étant constitué par des résidus d'Aβ1-15 et étant modifié par un acide palmitique lié aux extrémités N- et/ou C-terminales du peptide et étant reconstitué dans un liposome et lesdits sujets atteints du syndrome de Down n'ont pas encore développé de plaques associées à la protéine amyloïde dans le cerveau.

2. Peptide antigénique de la revendication 1, dans lequel le peptide Aβ1-15 est modifié par quatre acides palmitiques liés aux extrémités N- et/ou C-terminales du peptide.

3. Peptide antigénique de la revendication 1, dans lequel le peptide Aβ1-15 est modifié par deux acides palmitiques liés aux extrémités N- et/ou C-terminales du peptide.

4. Peptide antigénique destiné à être utilisé selon l'une quelconque des revendications 1 à 3, dans lequel ladite détérioration ou anomalie concerne une détérioration de la mémoire de reconnaissance, une détérioration de la mémoire associative contextuelle, une détérioration de l'apprentissage associatif, une détérioration de la mémoire déclarative pour des faits et événements, des détériorations épisodiques de la mémoire, un dysfonctionnement du langage, une déficience visio-spatiale, une diminution des fonctions exécutives, des changements de personnalité, des changements émotionnels, l'apraxie, des défaillances de l'exécution de tâches motrices apprises, des observations pyramidales et extrapyramidales, ainsi qu'une myoclonie ou des crises, ou bien une combinaison de ceux-ci.

5. Peptide antigénique destiné à être utilisé selon l'une quelconque des revendications 1 à 4, dans lequel ledit sujet atteint du syndrome de Down est
(a) un sujet d'âge jeune à moyen ; ou
(b) un sujet d'âge moyen ; ou
(c) un jeune sujet ; ou
(d) un sujet d'âge d'enfant ou
(e) un sujet de moins de 65 ans.

6. Peptide antigénique destiné à être utilisé selon l'une quelconque des revendications 1 à 5, dans lequel ladite utilisation
a) conduit à une amélioration et/ou à la restauration de la mémoire ; et/ou
b) conduit à une augmentation du maintien, ou à une restauration totale, de la capacité cognitive chez le sujet traité ; et/ou
c) conduit à un renforcement ou à une restauration de la mémoire et/ou de la capacité cognitive ; et/ou
d) n'induit pas d'effets secondaires indésirables.

7. Peptide antigénique destiné à être utilisé selon l'une quelconque des revendications 1 à 6 pour la prévention du développement de plaques associées à l'Aβ dans le cerveau.

8. Composition comprenant le peptide antigénique, tel que défini dans l'une quelconque des revendications 1 à 7, en une quantité efficace, d'un point de vue thérapeutique, conjointement avec un véhicule et/ou excipient acceptable d'un point de vue pharmaceutique, destinée à être utilisée dans le traitement et/ou le soulagement et/ou la prévention de détériorations ou d'anomalies de la mémoire et/ou cognitives chez des sujets atteints du syndrome de Down, dans laquelle lesdits sujets atteints du syndrome de Down n'ont pas encore développé de plaques associées à la protéine amyloïde dans le cerveau.

9. Composition destinée à être utilisée selon la revendication 8, comprenant un adjuvant.

10. Composition destinée à être utilisée selon la revendication 9, dans laquelle l'adjuvant est un lipide A.

11. Composition destinée à être utilisée selon la revendication 10, dans laquelle l'adjuvant est un lipide A détoxifié.

12. Composition destinée à être utilisée selon la revendication 9, dans laquelle l'adjuvant est un lipide A monophosphorylé ou diphosphorylé, l'alun, le phosphate de calcium, l'interleukine 1 et/ou des microcapsules de polysaccharides et protéines.

13. Peptide antigénique, destiné à être utilisé selon l'une quelconque des revendications 1 à 7, ou composition, destinée à être utilisée selon l'une quelconque des revendications 8 à 12, pour la prévention d'une neuro-dégénérescence chez un sujet atteint du syndrome de Down.

14. Peptide antigénique, destiné à être utilisé selon l'une quelconque des revendications 1 à 7, ou composition, destinée à être utilisée selon l'une quelconque des revendications 8 à 12, pour l'induction d'une réponse immunitaire contre une protéine bêta amyloïde chez un sujet atteint du syndrome de Down.


### SEQUENCE LISTING

<110> AC Immune S.A.
   PFEIFER, Andrea
   MUHS, Andreas
   MADANI, Rime
   VASILYEVICH, Pavel Belichenko
   MOBLEY, William
<120> Vaccine Therapy
<130> T2817 PCT BS
<150> PCT/US2011/052992
   <151> 2011-09-23
<160> 7
<170> PatentIn version 3.5
<210> 1
   <211> 15
   <212> PRT
   <213> Homo sapiens
<400> 1
   Asp Ala Glu Phe Arg His Asp Ser Gly Tyr Glu Val His His Gln
   1               5                   10                  15
<210> 2
   <211> 15
   <212> PRT
   <213> Mus musculus
<400> 2
   Asp Ala Glu Phe Gly His Asp Ser Gly Phe Glu Val Arg His Gln
   1               5                   10                  15
<210> 3
   <211> 15
   <212> PRT
   <213> Homo sapiens
<400> 3
   Asp Ala Glu Phe Arg His Asp Ser Gly Tyr Glu Val His Gln Lys
   1               5                   10                  15
<210> 4
   <211> 14
   <212> PRT
   <213> Homo sapiens
<400> 4
<210> 5
   <211> 14
   <212> PRT
   <213> Mus musculus
<400> 5
<210> 6
   <211> 16
   <212> PRT
   <213> Homo sapiens
<400> 6
   His Gln Lys Leu Val Phe Phe Ala Glu Asp Val Gly Ser Asn Lys Gly
   1               5                   10                  15
<210> 7
   <211> 16
   <212> PRT
   <213> Mus musculus
<400> 7
   His Gln Lys Leu Val Phe Phe Ala Glu Asp Val Gly Ser Asn Lys Gly
   1               5                   10                  15